(19) Europäisches Patentamt
European Patent Office
Office européen des brevets

(11) Veröffentlichungsnummer: **0 170 019**
**A1**

(12) # EUROPÄISCHE PATENTANMELDUNG

(21) Anmeldenummer: **85107420.3**

(22) Anmeldetag: **15.06.85**

(51) Int. Cl.[4]: **C 07 D 487/04**
A 61 K 31/40, C 07 F 9/65
//C07D205/08, C07D405/04,
C07F7/10, (C07D487/04, 209:00,
205:00)

(30) Priorität: **29.06.84 CH 3151/84**

(43) Veröffentlichungstag der Anmeldung:
**05.02.86 Patentblatt 86/6**

(84) Benannte Vertragsstaaten:
**BE CH DE FR GB IT LI NL SE**

(71) Anmelder: **F. HOFFMANN-LA ROCHE & CO. Aktiengesellschaft**

**CH-4002 Basel(CH)**

(72) Erfinder: **Furlenmeier, André, Dr.**
**Wettsteinallee 119**
**CH-4058 Basel(CH)**

(72) Erfinder: **Götschi, Erwin, Dr.**
**Tulpenweg 11**
**CH-4153 Reinach(CH)**

(74) Vertreter: **Notegen, Eric-André et al,**
**Grenzacherstrasse 124 Postfach 3255**
**CH-4002 Basel(CH)**

(54) **Carbapeneme mit Aethergruppe in 4-Stellung.**

(57) Die neuen Carbapeneme der Formel

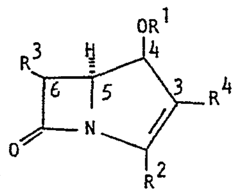

I

worin $R^1$ niederes Alkyl, Aryl oder Aryl-niederes Alkyl, $R^2$ Carboxy oder 5-Tetrazolyl, $R^3$ Wasserstoff oder einen für die 6-Stellung von Carbapenemen üblichen Substituenten und $R^4$ Wasserstoff oder einen für die 3-Stellung von Carbapenemen üblichen Substituenten bedeuten, herkömmliche, unter physiologischen Bedingungen hydrolysierbare Ester von Verbindungen der Formel I, worin $R^2$ Carboxy bedeutet, und pharmazeutisch annehmbare Salze davon und Hydrate dieser Stoffe besitzen wertvolle antibiotische Eigenschaften und können zur Bekämpfung oder Verhütung von Infektionskrankheiten verwendeten werden.

EP 0 170 019 A1

Croydon Printing Company Ltd.

0170019

F.HOFFMANN-LA ROCHE & CO.Aktiengesellschaft, Basel/Schweiz

RAN 4410/192

# Carbapeneme mit Aethergruppe in 4-Stellung

Die vorliegende Erfindung betrifft antibiotisch wirksame Carbapeneme der allgemeinen Formel

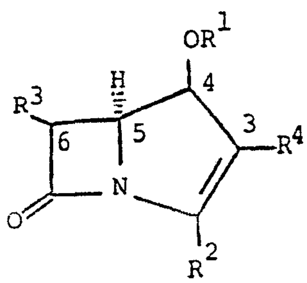

I

worin $R^1$ niederes Alkyl, Aryl oder Aryl-niederes Alkyl, $R^2$ Carboxy oder 5-Tetrazolyl, $R^3$ Wasserstoff oder eine für die 6-Stellung von Carbapenemen üblichen Substituenten und $R^4$ Wasserstoff oder einen für die 3-Stellung von Carbapenemen üblichen Substituenten bedeuten,

herkömmliche, unter physiologischen Bedingungen hydrolysierbare Ester von Verbindungen der Formel I, worin $R^2$ Carboxy bedeutet, pharmazeutisch annehmbare Salze davon und Hydrate dieser Stoffe.

In einer speziellen Ausführungsform betrifft die vorliegende Erfindung Verbindungen der allgemeinen Formel

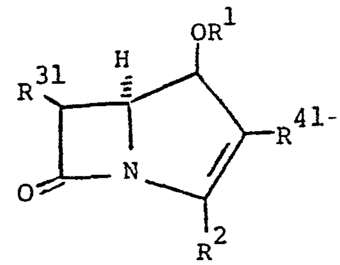

Ia

worin $R^1$ und $R^2$ obige Bedeutung besitzen, $R^{31}$ Wasserstoff, niederes Alkyl oder niederes Hydroxyalkyl, $R^{41}$ Wasserstoff oder die Gruppe $-S-(A)_m-(Q)_n-$ $-(A')_p-R^5$ (a), A und A' je niederes Alkylen oder niederes Alkenylen, m, n und p je die Zahl 0 oder 1, jedoch nicht alle gleichzeitig die Zahl 0, Q $(C_{3-6})$-Cycloalkylen, $(C_{3-6})$-Cycloalkenylen, Phenylen, 4- bis 7-gliedriges Heterocycloalkylen oder Heterocycloalkenylen oder 5- bis 6-gliedriges Heteroarylen und $R^5$ Wasserstoff, Halogen, Hydroxy, Nitro, Cyano, niederes Alkoxy, niederes Alkanoyl, niederes Alkanoyloxy, niederes Alkylthio, niederes Alkylsulfinyl, niederes Alkylsulfonyl, Carboxy, niederes Carbalkoxy, gegebenenfalls durch eine oder zwei niedere Alkylgruppen substituiertes Amino oder Carbamoyl, eine Amidgruppierung, eine Imidoylgruppierung oder eine Amidingruppierung bedeuten,

herkömmliche, unter physiologischen Bedingungen hydrolysierbare Ester von Verbindungen der Formel Ia, worin $R^2$ Carboxy bedeutet, pharmazeutisch annehmbare Salze davon und Hydrate dieser Stoffe.

Gegenstand der vorliegenden Erfindung sind ferner ein Verfahren zur Herstellung der vorstehend definierten Stoffe, Zwischenprodukte zu deren Herstellung, Arzneimittel auf der Basis dieser Stoffe und deren Herstellung, sowie die Verwendung dieser Stoffe bei der Behandlung oder Verhütung von Krankheiten und bei der Herstellung von antibiotisch wirksamen Mitteln.

Der in der vorliegenden Beschreibung verwendete Ausdruck "nieder" bezeichnet Reste und Verbindungen mit höchstens 7, vorzugsweise höchstens 4 Kohlenstoffatomen. Der Ausdruck "Alkyl" bezeichnet geradkettige oder verzweigte, gesättigte Kohlenwasserstoffreste, wie Methyl, Aethyl, Isopropyl und dgl. Der Ausdruck "Hydroxyalkyl" bezeichnet eine durch eine Hydroxygruppe substituierte Alkylgruppe. Der Ausdruck "Alkoxy" bezeichnet eine über ein Sauerstoffatom gebundene Alkylgruppe. Die Ausdrücke "Alkanoyl" und "Alkanoyloxy" bezeichnen geradkettige und verzweigte Fettsäurereste, wie Formyl, Acetyl, Acetoxy und dgl. Der Ausdruck "Halogen" bezeichnet die Halogene Fluor, Chlor, Brom und Jod.

Der Ausdruck "Aryl" bezeichnet carbocyclische, vorzugsweise monocyclische aromatische Gruppen, vorzugsweise gegebenenfalls durch Halogen, Trifluormethyl, Cyano, Nitro, Hydroxy, Carboxy, niederes Alkoxy oder niederes Alkyl substituiertes Phenyl.

Der Ausdruck "Alkylen" bezeichnet zweiwertige, geradkettige oder verzweigte, gesättigte Kohlenwasserstoffreste, wie Methylen, 1,2-Aethylen, Aethyliden und dgl. Der Ausdruck "Alkenylen" bezeichnet zweiwertige, geradkettige oder verzweigte, ungesättigte Kohlenwasserstoffreste, wie Propenylen und dgl. Der Ausdruck "Cycloalkylen" bezeichnet zweiwertige, gesättigte, cyclische Kohlenwasserstoffreste, wie Cyclopentylen, Cyclohexylen und dgl. Der Ausdruck "Cycloalkenylen" bezeichnet zweiwertige, ungesättigte, cyclische Kohlenwasserstoffreste, wie Cyclopentenylen, Cyclohexenylen und dgl.

Der Ausdruck "Heterocycloalkylen" bezeichnet zweiwertige, gesättigte heterocyclische Reste mit 1-4 Heteroatomen, wobei als Heteroatome insbesondere Stickstoff, Sauerstoff und Schwefel in Betracht kommen. Der Ausdruck "Heterocycloalkenylen" bezeichnet zweiwertige, partiell ungesättigte heterocyclische Reste mit 1-4 Heteroatomen, wobei

als Heteroatome insbesondere Sauerstoff, Stickstoff und Schwefel in Frage kommen. Der Ausdruck "Heteroarylen" bezeichnet zweiwertige, aromatische, heterocyclische Reste mit 1-4 Heteroatomen, wobei als Heteroatome insbesondere Stickstoff, Sauerstoff und Schwefel in Frage kommen. Die gesättigten und partiell ungesättigten heterocyclischen Reste sind vorzugsweise 5-gliedrig. Sie enthalten vorzugsweise eins bis drei Heteroatome, und zwar ein Sauerstoff-, Schwefel- oder Stickstoffatom und gegebenenfalls noch ein oder zwei Stickstoffatome. Die aromatischen heterocyclischen Reste enthalten als Heteroatome, falls sie 5-gliedrig sind, vorzugsweise ein Sauerstoff- oder Schwefelatom und gegebenenfalls noch ein oder zwei Stickstoffatome oder eins bis vier Stickstoffatome und, falls sie 6-gliedrig sind, vorzugsweise eins bis drei Stickstoffatome. Die heterocyclischen Reste können noch durch einen oder zwei für derartige Reste übliche Substituenten, beispielsweise durch niederes Alkyl, niederes Alkoxy, Hydroxy und Oxo, substituiert sein.

Der Ausdruck "Amidgruppierung" bezeichnet Reste der Formel

-NR-CO-R'     (b)

worin R und R' je Wasserstoff oder niederes Alkyl bedeuten.

Der Ausdruck "Imidoylgruppierung" bezeichnet Reste der Formel

-CR=NR'     (c)

worin R und R' obige Bedeutung besitzen.

Der Ausdruck "Amidingruppierung" bezeichnet Reste der Formeln

-NR-CR'=NR", -N=CR-NR'R" und -C(NRR')=NR"
(d) (e) (f)

worin R und R' obige Bedeutung besitzen und R" Wasserstoff oder niederes Alkyl bedeutet.

Der weiter unten verwendete Ausdruck "leicht abspaltbare Carboxylschutzgruppe" bezeichnet herkömmliche Gruppen, die unter milden Bedingungen, insbesondere reduktiv (z.B. hydrogenolytisch) oder hydrolytisch gespalten werden können. Geeignete Gruppen sind beispielsweise die 1-Aryl-niederen Alkylgruppen, wie die o- und p-Nitrobenzyl- oder p-Methoxy- benzylgruppen, die Benzhydrylgruppe, die 2,2,2-Trichloräthylgruppe, die niederen 2-Halogenalkylgruppen, wie die 2-Bromäthyl- und die 2-Jodäthylgruppe, die niederen 2-Trimethylsilylalkylgruppen, wie die 2-Trimethylsilyläthylgruppe, die Acetonylgruppe, die Allylgruppe und dgl.

Als leicht abspaltbare Carboxylschutzgruppen kommen auch die herkömmlichen, unter physiologischen Bedingungen, beispielsweise enzymatisch, abspaltbaren Gruppen in Frage. Beispiele solcher Gruppen sind die 1-niederen Alkanoyloxy-niederen Alkylgruppen, z.B. die Acetoxymethyl-, Pivaloyloxymethyl-, 1-Acetoxyäthyl- und die 1-Pivaloyloxyäthylgruppe; die 1-niederen Alkoxycarbonyloxy-niederen Alkylgruppen, z.B. die Methoxycarbonyloxymethyl-, 1-Aethoxycarbonyloxyäthyl- und die 1-Isopropoxycarbonyloxyäthylgruppe; die Lactonylgruppen, z.B. die Phthalidyl- und die Thiophthalidylgruppe; die niederen Alkoxymethylgruppen, z.B. die Methoxymethylgruppe; die niederen Alkanoylamidomethylgruppen, z.B. die Acetamidomethylgruppe; die Benzyl-, die Cyanomethyl- und die (2-Oxo-1,3-dioxol-4-yl)methylgruppe.

Der weiter unten verwendete Ausdruck "leicht abspaltbare N-Schutzgruppe" bezeichnet herkömmliche Gruppen, die unter milden Bedingungen, insbesondere reduktiv, z.B. hydrogenolytisch, abgespalten werden können. Als Beispiel

für eine solche Gruppe sei die p-Nitrobenzyloxycarbonyloxymethylgruppe erwähnt.

In einer bevorzugten Ausführungsform betrifft die vorliegende Erfindung Verbindungen der obigen Formel I, worin die 6-Stellung die (S)-Konfiguration aufweist, wenn $R^3$ eine von Wasserstoff verschiedene Bedeutung besitzt, und zwar insbesondere Verbindungen der allgemeinen Formel

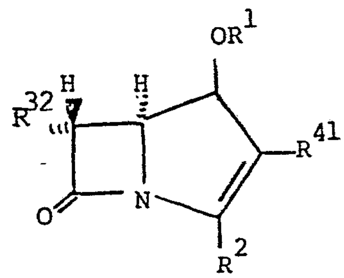

I'a

worin $R^1$, $R^2$ und $R^{41}$ obige Bedeutung besitzen, und $R^{32}$ niederes Alkyl oder niederes Hydroxyalkyl bedeutet.

$R^1$ bedeutet vorzugsweise niederes Alkoxy. $R^2$ bedeutet vorzugsweise Carboxy. Die bevorzugte Bedeutungsmöglichkeit von $R^3$ ist niederes 1-Hydroxyalkyl, insbesondere 1-Hydroxyäthyl.

Beispiele von erfindungsgemässen Verbindungen der Formel I sind:

(4S,5S,6S)-6-[(R)-1-Hydroxyäthyl]-4-methoxy-7-oxo-1-azabicyclo[3.2.0]hept-2-en-2-carbonsäure,

(4R,5S,6S)-6-[(R)-1-Hydroxyäthyl]-4-methoxy-7-oxo-1-azabicyclo[3.2.0]hept-2-en-2-carbonsäure,

(4R,5S,6S)-6-[(R)-1-Hydroxyäthyl]-4-methoxy-3-[(S)-3-pyrrolidinylthio]-7-oxo-1-azabicyclo[3.2.0]hept-2-en--2-carbonsäure,

(4R,5S,6S)-6-[(R)-1-Hydroxyäthyl]-3-[[(S)-1-(1-iminoäthyl)-3-pyrrolidinyl]thio]-4-methoxy-7-oxo-1-azabicyclo-[3.2.0]hept-2-en-2-carbonsäure,

(4R,5S,6S)-4-Aethoxy-6-[(R)-1-hydroxyäthyl]-3-[(S)-3-pyrrolidinylthio]-7-oxo-1-azabicyclo[3.2.0]hept-2-en-2-carbonsäure,

(4R,5S,6S)-4-Aethoxy-6-[(R)-1-hydroxyäthyl]-3-[[(S)-1-(1-iminoäthyl)-3-pyrrolidinyl]thio]-7-oxo-1-azabicyclo-[3.2.0]hept-2-en-2-carbonsäure,

(4R,5S,6S)-4-Aethoxy-6-[(R)-1-hydroxyäthyl]-3-(2-pyrimidinylthio)-7-oxo-1-azabicyclo[3.2.0]hept-2-en-2-carbonsäure,

(4R,5S,6S)-6-[(R)-1-hydroxyäthyl]-4-(1-isopentyloxy)-3-[(S)-3-pyrrolidinylthio]-7-oxo-1-azabicyclo[3.2.0]hept-2-en-2-carbonsäure und

(4R,5S,6S)-6-[(R)-1-hydroxyäthyl]-4-phenoxy-3-[(S)-3-pyrrolidinylthio]-7-oxo-1-azabicyclo[3.2.0]hept-2-en-2-carbonsäure.

Die Verbindungen der Formel I, die herkömmlichen, unter physiologischen Bedingungen hydrolysierbaren Ester von Verbindungen der Formel I, worin $R^2$ Carboxy bedeutet, die pharmazeutisch annehmbaren Salze davon und die Hydrate dieser Stoffe können erfindungsgemäss dadurch hergestellt werden, dass man

a) eine Verbindung der allgemeinen Formel

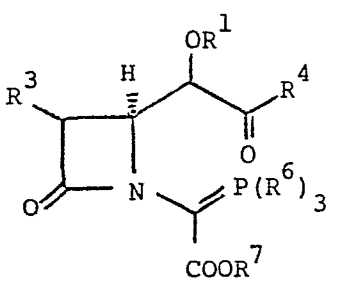

IIa

oder

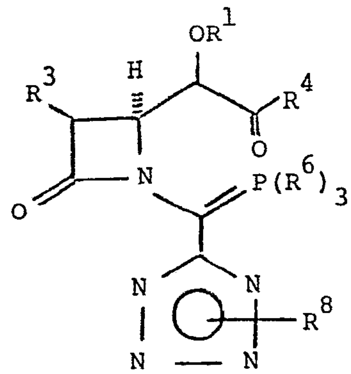

IIb

worin $R^1$, $R^3$ und $R^4$ die obige Bedeutung besitzen, $R^6$ niederes Alkyl, Phenyl oder p-Methoxyphenyl, $R^7$ eine leicht abspaltbare Carboxylschutzgruppe und $R^8$ eine leicht abspaltbare N-Schutzgruppe bedeuten, cyclisiert, oder

b) eine Verbindung der allgemeinen Formel

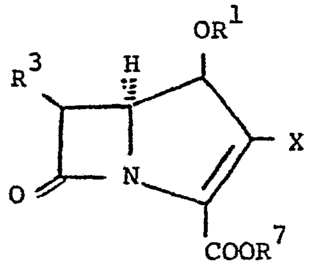

oder

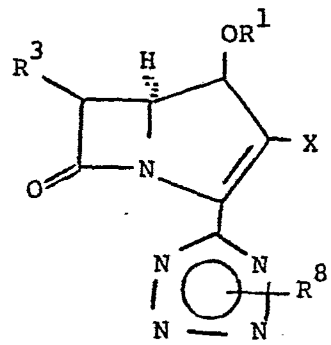

IIIb

IIIa

worin $R^1$, $R^3$, $R^7$ und $R^8$ die obige Bedeutung besitzen, und X eine Abgangsgruppe bedeutet,

entweder in Gegenwart einer Base mit einem Mercaptan der allgemeinen Formel

$$HS-(A)_m-(Q)_n-(A')_p-R^5 \qquad IV$$

worin A, A', Q, $R^5$, m, n und p die obige Bedeutung besitzen,

oder mit einem Salz eines solchen Mercaptans umsetzt, oder

c) eine Verbindung der allgemeinen Formel

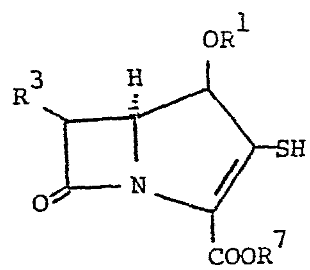

V

worin $R^1$, $R^3$ und $R^7$ die obige Bedeutung besitzen,

in Gegenwart einer Base oder ein Salz einer Verbindung der Formel V mit einer Verbindung der allgemeinen Formel

$$X-(A)_m-(Q)_n-(A')_p-R^5 \qquad VI$$

worin A, A', Q, $R^5$, m, n und p die obige Bedeutung besitzen und X' eine Abgangsgruppe bedeutet,

umsetzt, oder

d) eine Verbindung der allgemeinen Formel

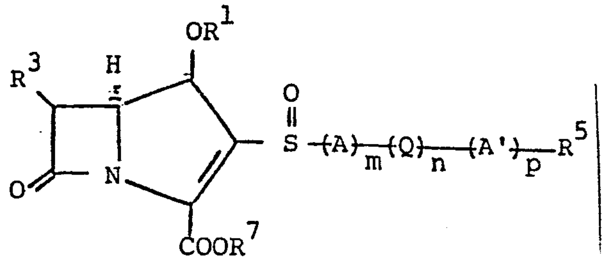

VII

worin A, A', Q, $R^1$, $R^3$, $R^5$, $R^7$, m, n und p obige Bedeutung besitzen,

reduziert, worauf man erwünschtenfalls den in einer erhaltenen Verbindung allfällig vorhandenen Substituenten in 3-Stellung in an sich bekannter Weise abwandelt und/oder eine erhaltenen Verbindung der Formel I entweder als einen unter physiologischen Bedingungen hydrolysierbaren Ester isoliert, oder allfällig vorhandene Schutzgruppen abspaltet und erwünschtenfalls eine so erhaltene Verbindung in ein pharmazeutisch annehmbares Salz und/oder Hydrat überführt.

Bei verschiedenen der obigen erfindungsgemässen Verfahren müssen allfällig vorhandene reaktionsfähige Amino- und/oder Hydroxylgruppen durch Schutzgruppen blockiert sein. Diese Fälle sind für den Fachmann ohne weiteres erkennbar, und auch die Auswahl der jeweils geeigneten Schutzgruppen bereitet ihm keine Schwierigkeiten.

Die Cyclisierung einer Verbindung der Formel IIa oder IIb gemäss Verfahrensvariante a) erfolgt unter Eliminierung eines Phosphinoxydes der allgemeinen Formel $(R^6)_3P=O$ und kann nach an sich bekannten und jedem Fachmann geläufigen Methoden durchgeführt werden. Die gewünschte Cyclisierung erfolgt dabei ziemlich leicht. Für den vorliegenden Zweck geeignete inerte Lösungsmittel sind beispielsweise aromatische Kohlenwasserstoffe, wie Benzol, Toluol und Xylol, Dioxan, halogenierte niedere Kohlenwasserstoffe, wie Methylenchlorid, Dimethylformamid und dgl. Die Cyclisierung kann in einem Temperaturbereich von etwa 0° bis etwa 150°C,

vorzugsweise in einem Bereich von Raumtemperatur bis zur Siedetemperatur der Reaktionsmischung (jedoch nicht über 150°C) durchgeführt werden.

Die Verbindung der Formel IIa oder IIb wird, wenn $R^4$ Wasserstoff bedeutet, zweckmässigerweise nicht isoliert, sondern im Reaktionsmedium, in welchen sie hergestellt worden ist, direkt cyclisiert.

Die Umsetzung einer Verbindung der Formel IIIa oder IIIb mit einer Verbindung der Formel IV in Gegenwart einer Base bzw. die Umsetzung einer Verbindung der Formel IIIa oder IIIb mit einem Salz einer Verbindung der Formel IV gemäss Verfahrensvariante b) kann ebenfalls nach an sich bekannten und jedem Fachmann geläufigen Methoden durchgeführt werden. Geeignete Abgangsgruppen sind beispielsweise die herkömmlichen organischen Sulfonsäurereste, beispielsweise die niederen Alkylsulfonyloxygruppen, wie die Methansulfonyloxygruppe, und die Arylsulfonyloxygruppen, wie die p-Toluolsulfonyloxygruppe, oder die niederen Alkylsulfinylgruppen, die Di(niederen Alkyl)phosphoryloxygruppen, beispielsweise die Diäthylphosphoryloxygruppe, die Diarylphosphoryloxygruppen, beispielsweise die Diphenylphosphoryloxygruppe, oder die Trifluormethansulfonyloxygruppe. Als Abgangsgruppen kommen jedoch auch die Halogenatome, beispielsweise Chlor-, Brom- und Jodatome in Frage. Geeignete inerte Lösungsmittel sind beispielsweise halogenierte niedere Kohlenwasserstoffe, wie Methylenchlorid, Acetonitril, Tetrahydrofuran, Dimethylformamid und dgl. Die Reaktion wird vorzugsweise bei Temperaturen zwischen etwa -80° und etwa 40°C durchgeführt. Geeignete Basen sind beispielsweise tertiäre Amine, wie Triäthylamin, Diisopropyläthylamin und 4-Dimethylaminopyridin, cyclische Amine, wie 1,5-Diazabicyclo[5.4.0]undec-5-en oder dgl., und Natriumhydrid.

Die Verbindungen der Formel IV können aber auch als Salze in die Reaktion eingesetzt werden, wobei diese Salze zweckmässigerweise unmittelbar vor der Umsetzung mit einer Verbindung der Formel IIIa bzw. IIIb in situ hergestellt werden. Als Salze kommen beispielsweise die Alkalimetallsalze, wie die Lithium- und Natriumsalze, oder auch Ammoniumsalze, wie die Triäthylammoniumsalze, in Frage. Wenn man diese Salze in situ herstellt, verwendet man vorzugsweise Basen, wie Butyllithium, Natriumhydrid, Triäthylamin und dgl.

Die Umsetzung einer Verbindung der Formel V mit einer Verbindung der Formel VI in Gegenwart einer Base bzw. die Umsetzung eines Salzes einer Verbindung der Formel V mit einer Verbindung der Formel VI gemäss Verfahrensvariante c) kann nach an sich bekannten Methoden durchgeführt werden. Die Abgangsgruppe X' ist beispielsweise ein herkömmlicher organischer Sulfonsäurerest, z.B. eine niedere Alkyl- oder Arylsulfonyloxygruppe, wie die Methansulfonyloxy- oder die p-Toluolsulfonyloxygruppe, oder ein Halogenatom, z.B. ein Chlor-, Brom- oder Jodatom. Die Reaktion wird vorzugsweise in einem polaren Lösungsmittel, wie N,N-Dimethylformamid, bei Temperaturen von -60° bis 0°C, insbesondere zwischen -20° und -60°C durchgeführt. Geeignete Basen sind beispielsweise tertiäre aliphatische Amine, wie Triäthylamin, und anorganische Basen, wie Kaliumcarbonat.

Die Verbindungen der Formel V können auch als Salze in die Reaktion eingesetzt werden, wobei diese Salze zweckmässigerweise unmittelbar vor der Umsetzung mit einer Verbindung der Formel VI in situ hergestellt werden. Geeignete Salze sind beispielsweise die Alkalimetallsalze, z.B. die Lithium- und Natriumsalze, und Ammoniumsalze, z.B. die Triäthylammoniumsalze. Wenn man solche Salze in situ herstellt, verwendet man vorzugsweise Basen, wie Butyllithium, Natriumhydrid und Triäthylamin.

Die Reduktion einer Verbindung der allgemeinen Formel VII gemäss Verfahrensvariante d) kann ebenfalls nach an sich bekannten und jedem Fachmann geläufigen Methoden erfolgen, beispielsweise mittels 2-Chlor- oder 2-Phenoxy--1,3,2-benzodioxophospholin in einem Gemisch aus Pyridin und einem aromatischen Kohlenwasserstoff, wie Benzol.

Die Abspaltung einer vorhandenen Carboxyl- oder N-Schutzgruppe aus einer gemäss Verfahrensvariante a), b), c) oder d) erhaltenen Verbindung erfolgt nach an sich bekannten und jedem Fachmann geläufigen Methoden.

Beispiele von Salzen von Verbindungen der Formel I sind die Alkalimetallsalze, wie die Natrium- und Kaliumsalze, die Ammoniumsalze, die Erdalkalimetallsalze, wie die Calciumsalze, die Salze mit organischen Basen, beispielsweise mit Aminen, wie N-Aethylpiperidin, Procain oder N,N-Dibenzyläthylendiamin, die Salze mit Aminosäuren, beispielsweise mit Arginin oder Lysin, und dgl.

Die Verbindungen der Formel I, welche einen basischen Substituenten aufweisen, bilden auch innere Salze und Salze mit organischen und anorganischen Säuren. Als Säuren kommen beispielsweise Mineralsäuren, wie Salzsäure, Schwefelsäure, Salpetersäure, Phosphorsäure und dgl., Sulfonsäuren, wie die Alkyl- und Arylsulfonsäuren, beispielsweise Aethansulfonsäure oder p-Toluolsulfonsäure, Essigsäure, Weinsäure, Maleinsäure, Zitronensäure, Benzoesäure, Salicyclsäure, Ascorbinsäure und dgl. in Frage.

Hydrate werden zumeist automatisch im Zuge des Herstellungsverfahrens oder als Folge hygroskopischer Eigenschaften eines zunächst wasserfreien Produktes erhalten. Zur gezielten Herstellung eines Hydrates kann ein ganz oder teilweise wasserfreies Produkt einer feuchten Atmosphäre ausgesetzt werden.

Die Verbindungen der Formel I enthalten mehrere asymmetrisch substituierte Kohlenstoffatome und können daher in verschiedenen Formen vorliegen. Die vorliegende Erfindung betrifft sowohl optisch einheitliche Formen, als auch Racemate und Mischungen von Diastereoisomeren.

Die als Ausgangsstoffe verwendeten Verbindungen der Formeln IIa und IIb können gemäss dem nachfolgenden Reaktionsschema I hergestellt werden.

Reaktionsschema I

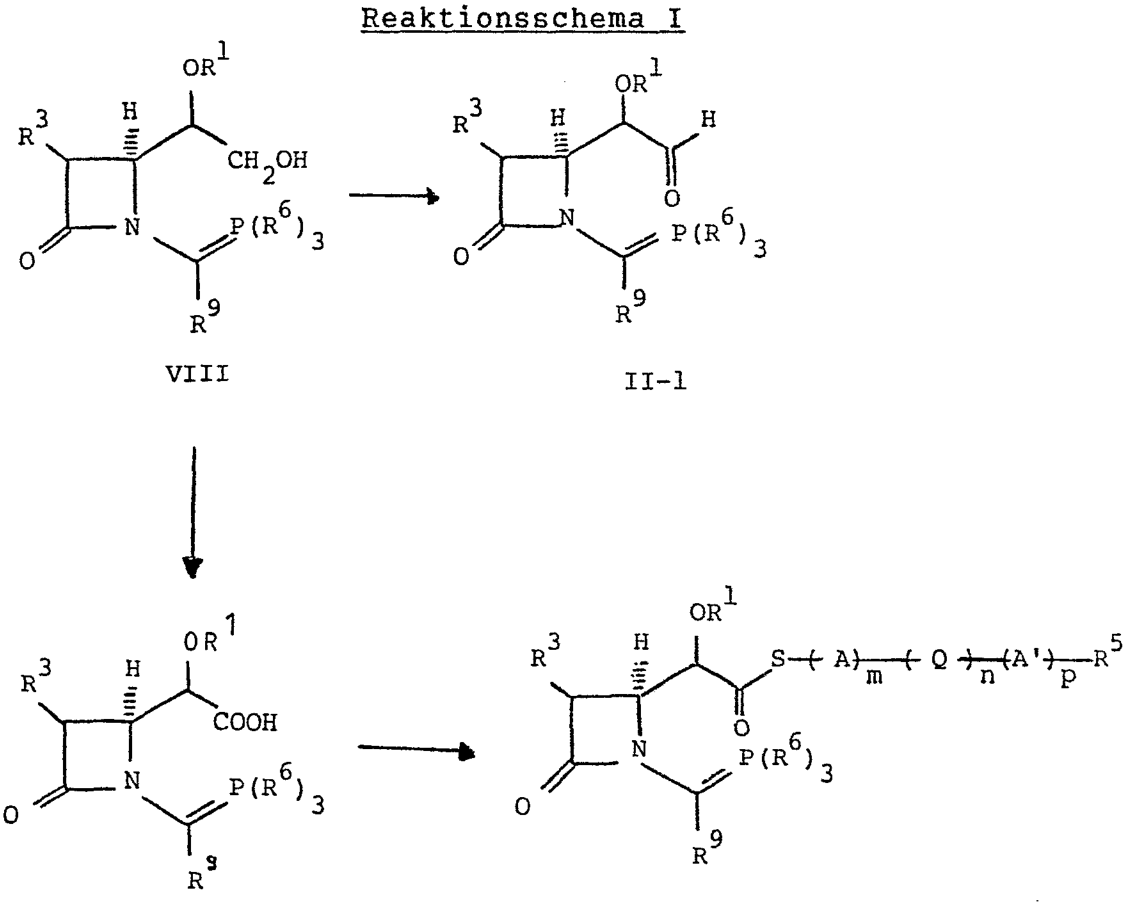

VIII II-1

IX II-2

$R^9$ bedeutet die Gruppe $-COOR^7$ oder 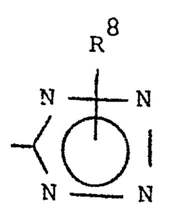 (g); $R^1$, $R^3$, $R^5$, $R^6$, $R^7$, $R^8$, A, A', Q, m, n und p besitzen obige Bedeutung.

Die Verbindungen der Formel II-1 können beispielsweise dadurch erhalten werden, dass man eine Verbindung der Formel VIII in an sich bekannter Weise oxydiert. Als Oxydationsmittel kann man beispielsweise ein 1:1-Gemisch von Essigsäureanhydrid und Dimethylsulfoxyd, Dicyclohexylcarbodiimid/Dimethylsulfoxyd oder Pyridindichromat/Methylenchlorid verwenden. Die Oxydation wird vorzugsweise bei Temperaturen von -20° bis etwa 100°C durchgeführt, wobei Temperaturen zwischen 0° und 30°C besonders bevorzugt werden.

Verbindungen der Formel II-2 können beispielsweise dadurch hergestellt werden, dass man zunächst eine Verbindung der Formel VIII in an sich bekannter Weise, beispielsweise unter Verwendung von Chromtrioxyd/Schwefelsäure/Aceton oder Kaliumpermanganat/Wasser, zur entsprechenden Carbonsäure der Formel IX oxydiert, diese Carbonsäure in an sich bekannter Weise in das entsprechende Carbonsäurechlorid überführt und dieses schliesslich mit einem Salz eines Mercaptans der allgemeinen Formel IV umsetzt. Die erwähnten Carbonsäurechloride können beispielsweise durch Reaktion mit einem Chlorierungsmittel, beispielsweise einem Phosphorsäure-di(niederen Alkyl)esterchlorid, wie Phosphorsäurediäthylesterchlorid, Thionylchlorid, Oxalylchlorid oder dgl., in Gegenwart eines tri (niederen Alkyl)amins, wie Triäthylamin, hergestellt werden. Das Salz eines Mercaptans der Formel IV ist vorzugsweise ein Alkalimetallsalz, beispielsweise ein Lithium- oder Natriumsalz, oder ein substituiertes Ammoniumsalz, beispielsweise ein Triäthylammoniumsalz. In einer bevorzugten Ausführungsform verwendet man ein entsprechendes Ammoniumsalz und stellt es in situ durch Umsetzen eines Mercaptans der Formel IV mit einem entsprechenden tertiären Amin her. Die obige Reaktion wird in einem inerten organischen Lösungsmittel, beispielsweise in Tetrahydrofuran, bei Temperaturen zwischen 0° und etwa 50°C durchgeführt.

Die als Ausgangsstoffe verwendeten Verbindungen der Formel III können beispielsweise gemäss dem nachfolgenden Reaktionsschema II hergestellt werden.

Reaktionsschema II

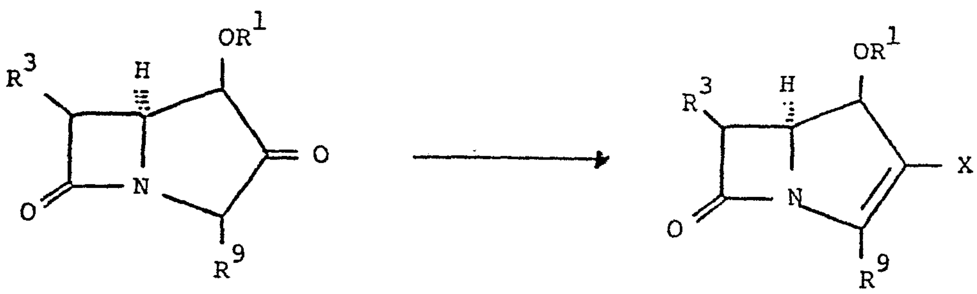

X

III

$R^1$, $R^3$ und $R^9$ besitzen obige Bedeutung; X bedeutet eine Abgangsgruppe.

Die Verbindungen der Formel III können nach an sich bekannten und jedem Fachmann geläufigen Methoden aus Verbindungen der Formel X hergestellt werden. Die Verbindungen der Formel III, worin X niederes Alkylsulfonyloxy, Arylsulfonyloxy, Dialkylphosphoryl, Diarylphosphoryl oder Trifluormethansulfonyloxy bedeutet, können dadurch hergestellt werden, dass man eine Verbindung der Formel IX mit einem geeigneten, entsprechenden Säurederivat umsetzt. Geeignete Säurederivate sind beispielsweise die Säurechloride und -anhydride. Geeignete inerte Lösungsmittel sind beispielsweise Methylenchlorid, Acetonitril, Tetrahydrofuran, Dimethylformamid und dgl. Die Reaktion wird zweckmässigerweise in Gegenwart einer Base, wie Triäthylamin, Diisopropyläthylamin oder 4-Dimethylaminopyridin und bei Temperaturen zwischen -78° und 20°C durchgeführt.

Die Verbindungen der Formel III, worin X niederes Alkylsulfinyl bedeutet, können beispielsweise dadurch hergestellt werden, dass man eine Verbindung der Formel Ia, worin $R^4$ niederes Alkylthio bedeutet, nach an sich bekannten und jedem Fachmann geläufigen Methoden zum entsprechenden Sulfoxyd oxydiert. Geeignete Oxydationsmittel sind beispielsweise Persäuren, wie m-Chlorperbenzoesäure, in einem inerten organischen Lösungsmittel, beispielsweise in Methylenchlorid, bei Temperaturen zwischen -20° und 30°C.

Die Verbindungen der Formel III, worin X niederes Alkylsulfinyl bedeutet, können auch ausgehend von Verbindungen der Formel I, worin $R^4$ Wasserstoff bedeutet, und zwar in Analogie zur weiter unten beschriebenen Herstellung von Verbindungen der Formel VII aus entsprechenden Verbindungen der Formel I-1a hergestellt werden.

Verbindungen der Formel III, worin X ein Halogenatom bedeutet, können ebenfalls nach an sich bekannten und jedem Fachmann geläufigen Methoden hergestellt werden, beispielsweise dadurch, dass man eine Verbindung der Formel III, worin X niederes Alkylsulfinyl bedeutet, mit einem Halogenierungsmittel, wie N-Brom- oder N-Jodsuccinimid, N-Brom- oder N-Jodacetamid oder mit einem Alkalimetalljodid in einem inerten Lösungsmittel, beispielsweise in Methylenchlorid, bei Temperaturen zwischen 0° und 70°C umsetzt.

Die Verbindungen der Formel III werden im allgemeinen nicht isoliert, sondern direkt weiterverarbeitet.

Die als Ausgangsstoffe verwendeten Verbindungen der Formel V können beispielsweise dadurch hergestellt werden, dass man eine Verbindung der Formel III, worin X niederes Alkylsulfinyl bedeutet, mit einem Alkalimetallhydrogensulfid, beispielsweise mit Natriumhydrogensulfid, in einem polaren, inerten Lösungsmittel, wie N,N-Dimethylformamid, bei Temperaturen zwischen -60° und -20°C umsetzt. Die Verbindungen der Formel V bzw. deren Alkalimetallsalze werden im allgemeinen nicht isoliert, sondern in situ wie weiter

oben beschrieben mit einem Alkylierungsmittel umgesetzt.

Die als Ausgangsstoffe verwendeten Verbindungen der Formel VII können gemäss dem nachfolgenden Reaktionsschema III hergestellt werden.

Reaktionsschema III

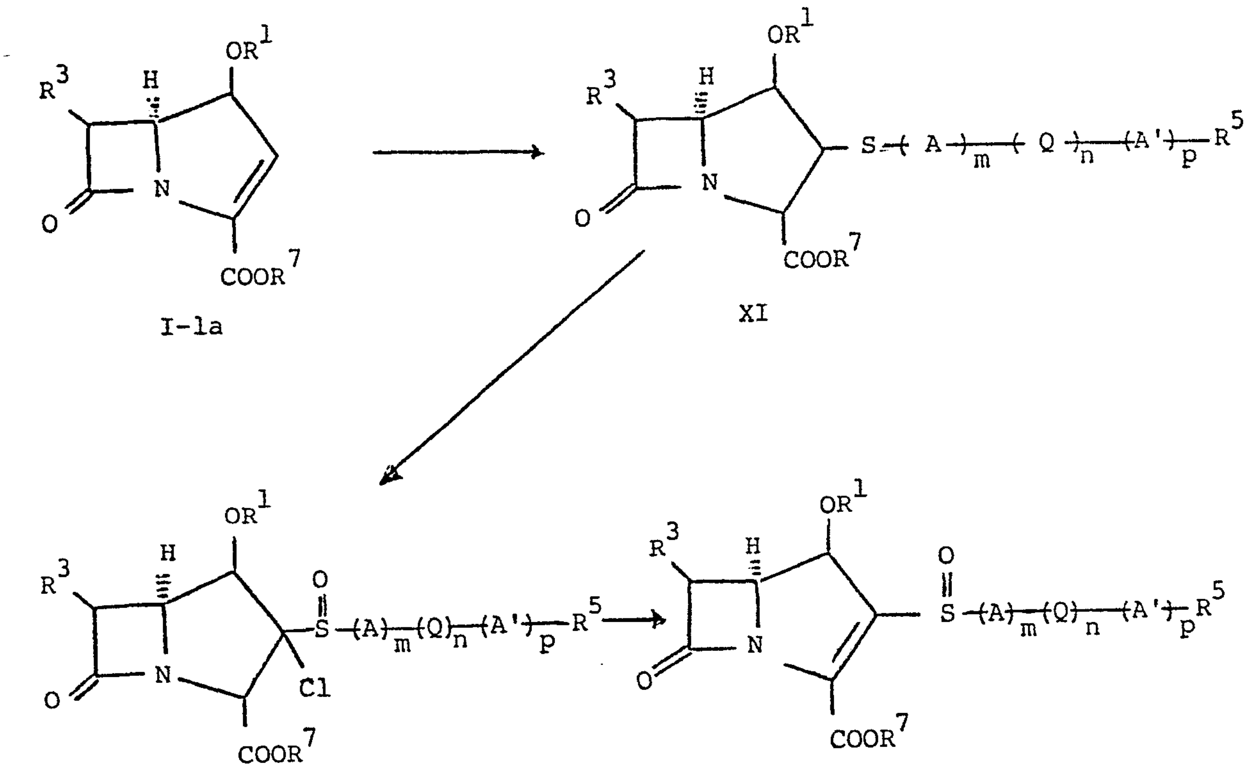

I-1a XI XII VII

$R^1$, $R^3$, $R^5$, $R^7$, A, A', Q, m, n und p besitzen obige Bedeutung.

Durch Reaktion einer Verbindung der Formel I-1a mit einem Mercaptan der Formel IV erhält man zunächst eine Verbindung der Formel XI. Diese Reaktion wird vorzugsweise in einem inerten organischen Lösungsmittel, wie Methylenchlorid, in Gegenwart einer Base, wie 1,5-Diazabicyclo[5.4.0]-undec-5-en oder Triäthylamin, in einem Bereich von etwa -50° bis +30°C durchgeführt. Eine so erhaltene Verbindung der Formel XI kann dann durch Oxydation mit Jodbenzoldichlorid in einem Gemisch aus Chloroform, Pyridin und Wasser

bei Temperaturen von 0° bis 60°C in ein Sulfoxyd der allgemeinen Formel XII übergeführt werden. Durch Eliminierung eines Aequivalentes Chlorwasserstoff aus der letztgenannten Verbindung erhält man dann eine Verbindung der Formel VII. Die Eliminierung kann beispielsweise in einem inerten Lösungsmittel, wie Aethylacetat, in Gegenwart einer Base, wie 1,5-Diazabicyclo[5.4.0]undec-5-en, bewerkstelligt werden.

Die Verbindungen der Formel VIII können gemäss dem nachfolgenden Reaktionsschema IV hergestellt werden.

Reaktionsschema IV

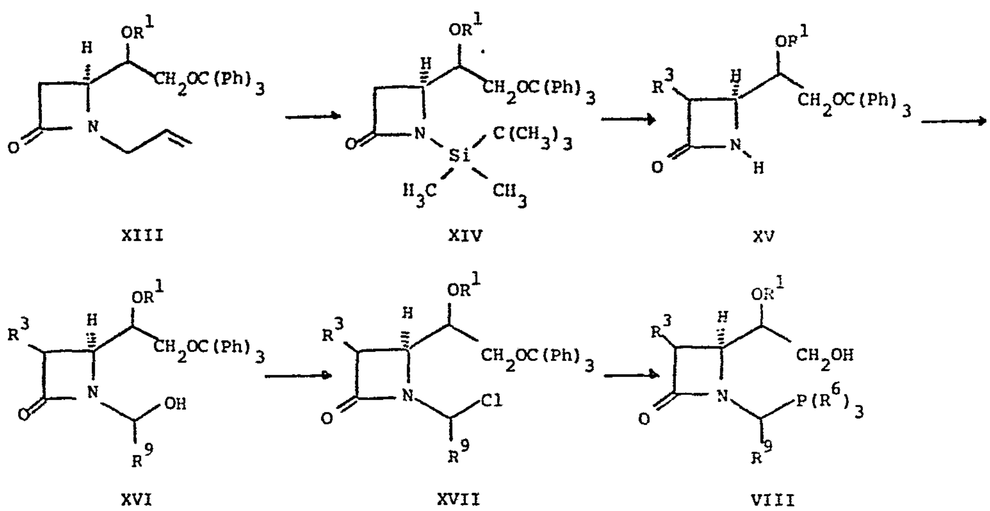

XIII XIV XV

XVI XVII VIII

$R^1$, $R^3$, $R^6$ und $R^9$ besitzen obige Bedeutung; Ph bedeutet Phenyl.

Die Verbindungen der Formel XIV werden dadurch hergestellt, dass man aus einer Verbindung der Formel XIII die Allylschutzgruppe abspaltet und anschliessend das ß-Lactamstickstoffatom mit der t-Butyldimethylsilylgruppe schützt. Die Abspaltung der Allylgruppe aus Verbindungen der Formel XIII erfolgt durch Isomerisierung der Allylgruppe zu einem E/Z-Gemisch der entsprechenden N-Propenylverbindungen und anschliessender oxydativer Spaltung der Propenylgruppe. Die Isomerisierung kann beispielsweise mit einer starken Base, wie Kalium-t-butylat in Dimethylformamid bei Temperaturen zwischen 0° und 40°C durchgeführt werden. Die Spaltung der Propenylgruppe kann beispielsweise mit Kaliumpermanganat in einem Gemisch aus Pyridin und Wasser und erwünschtenfalls einem inerten Lösungsmittel, wie Dimethoxyäthan, Tetrahydrofuran oder Aceton, bei Temperaturen zwischen 0° und 50°C durchgeführt werden. Durch Umsetzen einer so erhaltenen Verbindung mit t-Butyldimethylchlorsilan in Dimethylformamid in Gegenwart von Triäthylamin erhält man dann eine Verbindung der Formel XIV.

Eine Verbindung der Formel XIV kann dann beispielsweise mit Lithiumdiisopropylamid in Tetrahydrofuran/Hexan, in das entsprechende Lithiumenolat übergeführt werden, und dieses kann dann bei Temperaturen von -50° bis -120°C mit einem entsprechenden, den gewünschten Rest $R^3$ liefernden, elektrophilen Reagens zu einer Verbindung der Formel XV umgesetzt werden. Geeignete elektrophile Reagenzien sind beispielsweise die den Rest $R^3$ liefernden Halogenide oder Aldehyde. Bei dieser Umsetzung erhält man gewöhnlich Diastereomerengemische, welche erwünschtenfalls mit den üblichen Trennmethoden, beispielsweise Chromatographie an Kieselgel oder fraktionierte Kristallisation, aufgetrennt werden können. Man kann aber ohne weiteres die Synthese auch mit einem Diastereomerengemisch fortsetzen und die Trennung der Diastereomeren gewünschtenfalls zu einem späteren Zeitpunkt vornehmen.

Durch Abspalten der Silylschutzgruppe aus einer so erhaltenen Verbindung, beispielsweise mit Ammoniumfluorid in

Methanol, erhält man eine Verbindung der Formel XV.

Aus Verbindungen der Formel XV können nach an sich bekannten und jedem Fachmann geläufigen Methoden die Verbindungen der Formel XVI hergestellt werden. Verbindungen der Formel XVI, worin $R^9$ die Gruppe $-COOR^7$ bedeutet, können beispielsweise hergestellt werden, indem man eine Verbindung der Formel XV mit Glyoxylsäuremonohydrat in Dimethylformamid in Gegenwart einer Base wie Diisopropyläthylamin umsetzt und in der erhaltenen Verbindung die freie Carboxylfunktion mit einem geeigneten Alkylierungsmittel, wie p-Nitrobenzylbromid oder Pivalinsäurejodmethylester blockiert. Die Verbindungen der Formel XVI, worin $R^9$ die obige Gruppe (g) bedeutet, können dadurch hergestellt werden, dass man eine Verbindung der Formel XV mit einem Aldehyd der allgemeinen Formel

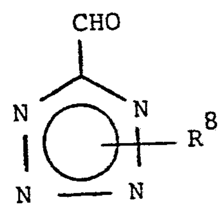

XXVIII

worin $R^8$ obige Bedeutung besitzt,
bzw. mit einem Hydrat desselben in Dimethylformamid in Gegenwart von Diisopropyläthylamin umsetzt.

Durch Reaktion einer Verbindung der Formel XVI mit Thionylchlorid in einem inerten organischen Lösungsmittel, wie Tetrahydrofuran, in Gegenwart einer Base, wie Pyridin oder 2,6-Lutidin, bei Temperaturen zwischen -30° und 20°C erhält man die entsprechende chlorierte Verbindung der Formel XVII.

Durch Reaktion einer Verbindung der Formel XVII mit einem substituierten Phosphin der allgemeinen Formel $P(R^6)_3$, vorzugsweise Triphenylphosphin, in an sich bekannter Weise, d.h. in einem aprotischen Lösungsmittel, wie

Dioxan, Tetrahydrofuran oder Dimethylformamid, bei Temperaturen zwischen 0° und 60°C erhält man das entsprechende Phosphoran. Aus der so erhaltenen Verbindung können die Verbindungen der Formel VIII durch säurekatalysierte Abspaltung der Triphenylmethylschutzgruppe, beispielsweise durch Erwärmen in wässriger Essigsäure, erhalten werden.

Die Verbindungen der Formel X können gemäss dem nachfolgenden Reaktionsschema V hergestellt werden.

Reaktionsschema V

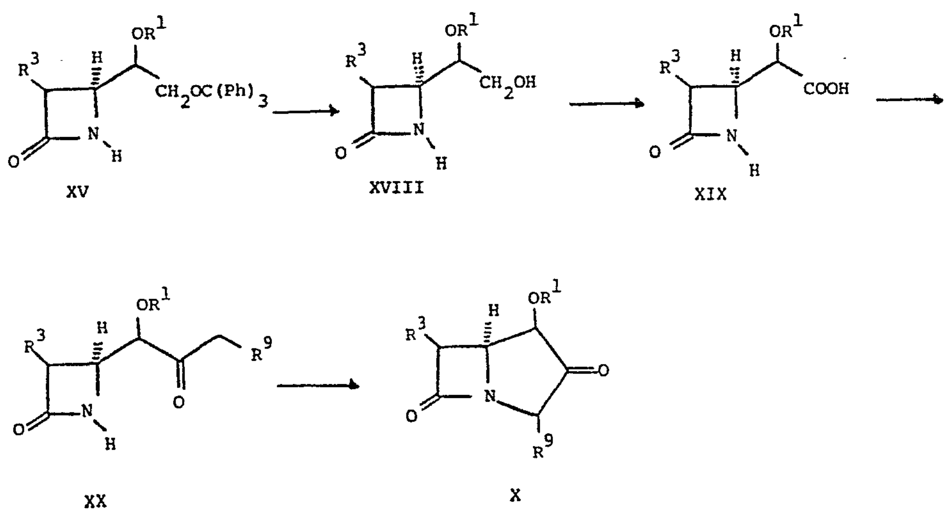

XV XVIII XIX

XX X

$R^1$, $R^3$, $R^9$ und Ph besitzen obige Bedeutung.

Durch säurekatalysierte Abspaltung der Triphenylmethylschutzgruppe, beispielsweise durch Erwärmen in wässriger Essigsäure, erhält man aus einer Verbindung der Formel XV eine solche der Formel XVIII. Diese kann nach an sich bekannten und jedem Fachmann geläufigen Oxydationsmethoden in

eine entsprechende Verbindung der Formel XIX übergeführt werden. Geeignete Oxydationsmittel sind beispielsweise Kaliumpermanganat/Wasser und Chromtrioxyd/Schwefelsäure/-Aceton.

Die Verbindungen der Formel XX, worin $R^9$ die Gruppe $-COOR^7$ bedeutet, können dadurch hergestellt werden, dass man zunächst eine Verbindung der Formel XIX durch Behandeln in einem inerten Lösungsmittel, wie Methylenchlorid, Tetrahydrofuran, Chloroform oder Toluol, mit einem Chlorierungsmittel, wie Thionylchlorid, Oxalylchlorid oder Phosphoroxychlorid, in das entsprechende Säurechlorid überführt, dieses gemäss der von Y. Oikawa et al. in J. Org. Chem. 43, 2088 (1978) beschriebenen Methode mit dem cyclischen Isopropylidenester der Malonsäure (Meldrumsäure) in Methylenchlorid/Pyridin umsetzt und das erhaltene Produkt schliesslich in einem inerten Lösungsmittel, wie Tetrahydrofuran, in Gegenwart eines Alkohols der allgemeinen Formel $R^7$-OH, worin $R^7$ obige Bedeutung besitzt, erhitzt.

Eine Verbindung der Formel XX, worin $R^9$ die Gruppe $-COOR^7$ bedeutet, kann auch dadurch hergestellt werden, dass man eine Verbindung der Formel XIX zunächst mit 1,1--Carbonyldiimidazol in einem inerten Lösungsmittel, wie Tetrahydrofuran, bei Temperaturen von 0° bis 40°C in das entsprechende Carbonsäureimidazolid überführt und dieses in situ mit einem Ueberschuss (1,1-3 Aequivalenten) an einer Verbindung der allgemeinen Formel $(R^7OOCCH_2COO)_2Mg$, worin $R^7$ obige Bedeutung besitzt, bei Temperaturen von 0° bis 40°C umsetzt.

Die Verbindungen der Formel XX, worin $R^9$ die obige Gruppe (g) bedeutet, können nach an sich bekannten Methoden dadurch hergestellt werden, dass man eine Verbindung der Formel XIX zunächst wie oben beschrieben in das entsprechende Säurechlorid überführt und dieses mit dem Lithiumsalz eines Esters der allgemeinen Formel

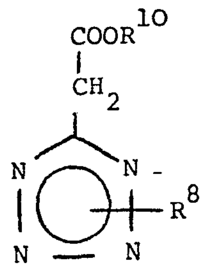

XXIX

worin $R^8$ obige Bedeutung besitzt und $R^{10}$ tert.Butyl bedeutet,

umsetzt und in der erhaltenen Verbindung die Gruppe $-COOR^{10}$ durch Esterspaltung und Decarboxylierung abspaltet.

Je nach angewendeten Reaktionsbedingungen ist es zweckmässig, das ß-Lactamstickstoffatom zu blockieren, wobei man vorzugsweise die t-Butyldimethylsilylschutzgruppe verwendet. Die Verbindungen der Formel XX können schliesslich nach an sich bekannten und jedem Fachmann geläufigen Methoden in Verbindungen der Formel X übergeführt werden. Man kann beispielsweise eine Verbindung der Formel XX mit einem Diazo-Uebertragungsreagens, wie p-Toluolsulfonylazid, in einem inerten Lösungsmittel, wie Aethylacetat, Acetonitril oder Toluol, und in Gegenwart einer Base, wie Triäthylamin, bei Temperaturen von 0° bis 40°C zunächst in die entsprechende Diazo-Verbindung überführen und diese dann durch Erhitzen in einem inerten Lösungsmittel, wie Benzol, Toluol oder Tetrahydrofuran, bei Temperaturen zwischen 50° und 110°C und in Gegenwart eines Katalysators, wie Rhodium(II)-acetat, Rhodium(II)octanoat, Palladiumdiacetat oder Kupfersulfat, in eine entsprechende Verbindung der Formel X überführen.

Die als Ausgangsstoffe verwendeten Verbindungen der Formel XIII können beispielsweise gemäss dem nachfolgenden Reaktionsschema VI hergestellt werden.

Reaktionsschema VI

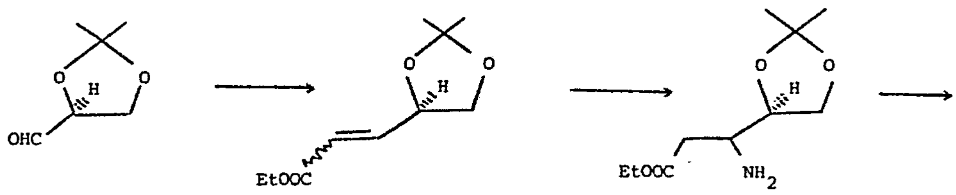

XXI XXII XXIII

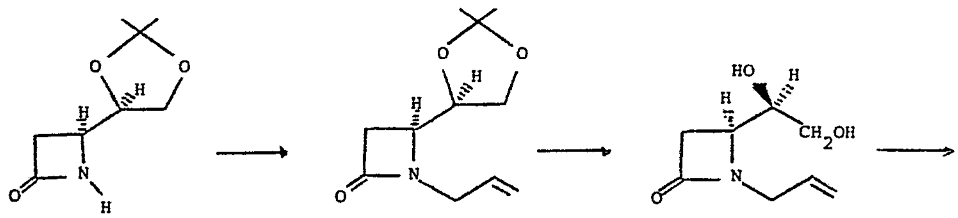

XXIV XXV XXVI

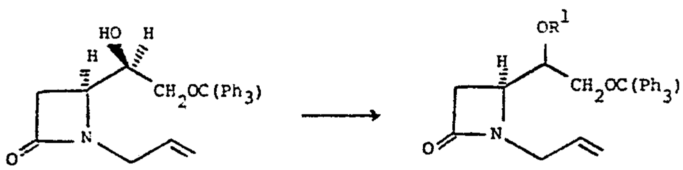

XXVII XIII

$R^1$ und Ph besitzen obige Bedeutung; Et bedeutet Aethyl.

2,3-O-Isopropyliden-L-glyceraldehyd der Formel XXI kann durch Umsetzen mit Phosphoressigsäuretriäthylester in einem Gemisch aus Methylenchlorid und 28-proz. Natronlauge in Gegenwart von Benzyltriäthylammoniumchlorid in die Verbindung der Formel XXII übergeführt werden (man erhält das Produkt als Gemisch der E- und Z-Isomeren). Durch Addition von Ammoniak an die in der Verbindung der Formel XXII vorhandene Doppelbindung erhält man die Verbindung der Formel XXIII als (3S/3R)-Epimerengemisch. Die Reaktion wird dabei in mit Ammoniak gesättigtem Alkohol bei Temperaturen zwischen -20° und 100°C, vorzugsweise zwischen 0° und 30°C, unter Normaldruck oder erhöhtem Druck (Druckgefäss) durchgeführt. Unter den bevorzugten Reaktionsbedingungen wird das gewünschte 3S-Epimere als Hauptprodukt gebildet. Durch Cyclisieren einer Verbindung der Formel XXIII in an sich bekannter Weise, beispielsweise durch Umsetzen mit Trimethylchlorsilan in Gegenwart von Triäthylamin und anschliessendem Behandeln mit einem Aequivalent Aethylmagnesiumbromid, erhält man die Verbindung der Formel XXIV. Durch Umsetzen mit Allylbromid, beispielsweise in Tetrahydrofuran und in Gegenwart von Kaliumhydroxyd und einer katalytischen Menge von Benzyltriäthylammoniumchlorid, erhält man aus einer Verbindung der Formel XXIV eine solche der Formel XXV. Durch säurekatalysierte Hydrolyse, beispielsweise mittels p-Toluolsulfonsäure in einem Gemisch aus Tetrahydrofuran und Wasser, erhält man aus der letztgenannten Verbindung die Verbindung der Formel XXVI. Durch Umsetzen dieser Verbindung mit einem Aequivalent Triphenylchlormethan in Pyridin erhält man die Verbindung der Formel XXVII. Die Verbindung der Formel XXVII kann nach allgemein bekannten Methoden in den entsprechenden epimeren Alkohol übergeführt werden. So erhält man durch Behandeln der Verbindung der Formel XXVII mit Azodicarbonsäurediisopropylester in Gegenwart von Triphenylphosphin und Benzoesäure in Tetrahydrofuran ein Benzoat, aus dem durch alkalisches Verseifen oder Umesterung, beispielsweise mit Kaliumhydroxyd in Methanol, der epimere Alkohol (S)-1-Allyl-4-[(S)-1-hydroxy-2-(triphenylmethoxy)äthyl]-2-azetidinon hergestellt werden kann. Man kann die freie Hydroxygruppe in einem Alkohol der Formel XXVII oder in dessen Epimerenalkohol nach an sich bekannten und jedem Fachmann geläufigen Methoden veräthern. Man kann beispielsweise einen solchen Alkohol mit einem Halogenid der allgemeinen Formel $R^1$-Y, worin Y Halogen bedeutet und $R^1$ obige Bedeutung besitzt, in Gegenwart einer starken Base, wie Natriumhydrid, in einem polaren, aprotischen Lösungsmittel wie Dimethylformamid, zu einer Verbindung der allgemeinen Formel XIII veräthern.

Wie bereits eingangs erwähnt, besitzen die erfindungsgemässen Verbindungen wertvolle antibiotische Eigenschaften; sie sind insbesondere gegen gram-positive und gram-negative Bakterien, wie beispielsweise gegen Staphylokokken, Streptokokken, Salmonellen und Escherichia coli, wirksam. Sie besitzen darüber hinaus auch ß-Lactamase hemmende Eigenschaften und zeichnen sich durch eine überraschend hohe Stablität gegenüber dem Enzym Dehydropeptidase I aus, das die Hydrolyse von Carbapenemen katalysiert.

Die minimalen Hemmkonzentrationen (MHK) der (4S,5S,6S)-6-[(R)-1-Hydroxyäthyl]-4-methoxy-7-oxo -1-azabicyclo-[3.2.0]hept-2-en-2-carbonsäure (Verbindung A), der (4R,5S,-6S)-6-[(R)-1-Hydroxyäthyl]-3-[[(S)-1-(1-iminoäthyl) -3-pyrrolidinyl]thio]-4-methoxy-7-oxo -1-azabicyclo[3.2.0]-hept-2-en -2-carbonsäure (Verbindung B), der (4R,5S,6S)-4-Aethoxy-6-[(R)-1-hydroxyäthyl]-3-[(S) -3-pyrrolidinylthio]-7-oxo-1-azabicyclo[3.2.0]hept-2-en -2-carbonsäure (Verbindung C) und der (4R,5S,6S)-4-Aethoxy-6-[(R)-1-hydroxyäthyl]-3-[[(S)-1 -(1-iminoäthyl)-3-pyrrolidinyl]-thio]-7-oxo-1 -azabicyclo[3.2.0]hept-2-en-2-carbonsäure (Verbindung D) gegen eine Reihe pathogener Mikroorganismen wurden auf DST-Agar (Diagnostic Sensitivity Test Agar) bestimmt. Die erhaltenen Resultate sind in der nachfolgen-

den Tabelle aufgeführt:

Tabelle

| Organismus | MHK (µg/ml) der Verbindung A | B | C | D |
|---|---|---|---|---|
| E. coli 1346 | 0,5 | 0,4 | 0,5 | 1 |
| " 25922 | 1 | 0,8 | 0,5 | 2 |
| " TEM 1* | 1 | 0,4 | 0,5 | 0,5 |
| " 1527 E* | 0,5 | 0,4 | 0,25 | 1 |
| Klebs. pneumoniae 418* | 0,5 | 0,4 | 2 | 2 |
| Prot. vulgaris 1028* | 4 | 12,5 | 32 | 16 |
| Prot. mirabilis 2117* | 4 | 12,5 | 16 | 16 |
| Prot. mirabilis 29H | 8 | 25 | 32 | 32 |
| Strept. faecalis 6 | 32 | 12,5 | 32 | 32 |
| Staph. aureus 6538 | 0,5 | 0,1 | 0,25 | 0,25 |
| " 887* | 1 | 0,2 | 0,5 | 0,5 |
| Serratia marcescens 803/15* | 2 | 3,1 | 4 | 8 |
| Serratia marcescens 69438 | 2 | 3,1 | 8 | 8 |
| Klebs. aerogenes 1082E* | 1 | 0,8 | 2 | 2 |
| Enterobacter cloacae 908* | 1 | 0,8 | 1 | 4 |
| Enterobacter cloacae 15M* | 1 | 0,8 | 1 | 4 |
| Salmonella typh. BA | 1 | 0,8 | 0,5 | 2 |
| Pseudomonas aer. 1559E* | >32 | 25 | 64 | 64 |
| " BA* | 32 | 25 | 32 | 64 |
| Acinetobacter anitr. 5I-156 | 2 | 0,4 | 1 | 2 |

Die ß-Lactamase produzierenden Organismen sind mit einem * bezeichnet.

Die erfindungsgemässen Stoffe können als Heilmittel, z.B. in Form pharmazeutischer Präparate zur enteralen oder parenteralen Applikation, Verwendung finden. Die erfindungsgemässen Stoffe können beispielsweise peroral, z.B. in Form von Tabletten, Lacktabletten, Dragées, Hart- und Weichgelatinekapseln, Lösungen, Emulsionen oder Suspensionen, rektal, z.B. in Form von Suppositorien, oder parenteral, z.B. in Form von Injektionslösungen, verabreicht werden.

Die Herstellung der pharmazeutischen Präparate kann in jedem Fachmann geläufiger Weise dadurch erfolgen, dass man die erfindungsgemässen Stoffe, gegebenenfalls in Kombination mit anderen therapeutisch wertvollen Stoffen, zusammen mit geeigneten, nicht-toxischen, inerten, therapeutisch verträglichen festen oder flüssigen Trägermaterialien und gegebenenfalls den üblichen pharmazeutischen Hilfsstoffen in eine galenische Darreichungsform bringt.

Als solche Trägermaterialien eignen sich sowohl anorganische als auch organische Trägermaterialien. So kann man für Tabletten, Lacktabletten, Dragées und Hartgelatinekapseln beispielsweise Lactose, Maisstärke oder Derivate davon, Talk, Stearinsäure oder deren Salze als Trägermaterialien verwenden. Für Weichgelatinekapseln eignen sich als Träger beispielsweise pflanzliche Oele, Wachse, Fette und halbfeste und flüssige Polyole (je nach Beschaffenheit des Wirkstoffes sind jedoch bei Weichgelatinekapseln keine Träger erforderlich). Zur Herstellung von Lösungen und Sirupen eignen sich als Trägermaterialien beispielsweise Wasser, Polyole, Saccharose, Invertzucker und Glucose. Für Injektionslösungen eignen sich als Trägermaterialien beispielsweise Wasser, Alkohole, Polyole, Glycerin und pflanzliche Oele. Für Suppositorien eignen sich als Trägermaterialien beispielsweise natürliche oder gehärtete Oele, Wachse, Fette und halbflüssige oder flüssige Polyole.

Als pharmazeutische Hilfsstoffe kommen die üblichen Konservierungsmittel, Lösungsvermittler, Stabilisierungsmittel, Netzmittel, Emulgiermittel, Süssmittel, Färbemittel, Aromatisierungsmittel, Salze zur Veränderung des osmotischen Druckes, Puffer, Ueberzugsmittel und Antioxydantien in Frage.

Die Verbindungen der Formel I und ihre Salze bzw. Hydrate kommen insbesondere für die parenterale Applikation in Betracht und werden zu diesem Zweck vorzugsweise als Lyophilisat oder Trockenpulver zur Verdünnung mit üblichen Trägern, wie Wasser oder isotonischer Kochsalzlösung, zur Verfügung gestellt.

Die pharmazeutischen Präparate können die erfindungsgemässen Stoffe in Mengen von etwa 25-1000 mg, vorzugsweise 50-500 mg, pro Einzeldosierungsform enthalten. Für die Prophylaxe und Therapie von Infektionskrankheiten kommt für den Erwachsenen eine tägliche Dosis von etwa 0,05 g bis etwa 4 g, insbesondere etwa 0,1 g bis etwa 2 g in Betracht.

Die nachfolgenden Beispiele sollen die vorliegende Erfindung näher erläutern, ihren Umfang jedoch in keiner Weise beschränken. Sämtliche Temperaturen sind in Celsiusgraden angegeben.

## Beispiel 1

a) Ein auf 3° vorgekühltes Gemisch aus 20 ml Methylenchlorid, 20 ml 28-proz. wässriger Natronlauge und 0,6 g Benzyltriäthylammoniumchlorid versetzt man unter starkem Rühren innert 10 Minuten mit einer Lösung von 13,0 g 2,3-O-Isopropyliden-L-glycerinaldehyd und 22,4 g Phosphonoessigsäure-triäthylester in 60 ml Methylenchlorid, wobei die Temperatur auf 20° ansteigt. Man rührt noch 30 Minuten bei 20°, trennt die Wasserphase ab, wäscht die organische Phase viermal mit je 20 ml Wasser neutral, trocknet über Natriumsulfat und dampft ein. Das verbleibende Oel wird über eine 20 cm lange Vigreux-Kolonne destilliert. Man erhält (R)-2,2-Dimethyl-1,3-dioxolan-4-acrylsäureäthylester vom Siedepunkt 54-58° (0,2 mm), als Gemisch der E- und Z-Isomeren (Verhältnis ca. 3:1).

b) Eine Lösung von 53,8 g (R)-2,2-Dimethyl-1,3-dioxolan-4-acrylsäureäthylester (E/Z-Gemisch) in 440 ml Aethanol wird bei einer Temperatur von 15° mit trockenem, gasförmigem Ammoniak gesättigt. Man lässt während 110 Stunden bei Raumtemperatur stehen, dampft dann im Vakuum ein und destilliert das verbleibende Oel über eine 10 cm lange Vigreux-Kolonne. Man erhält 3-Amino-3-(2,2-dimethyl-1,3-dioxolan-4-yl)propionsäureäthylester vom Siedepunkt 69-74° (0,15 mm) als Diastereomerengemisch (3S:3R ca. 88:12).

c) Eine auf 0° vorgekühlte Lösung von 187,2 g 3-Amino-3-(2,2-dimethyl-1,3-dioxolan-4-yl)propionsäureäthylester (Diastereomerengemisch) in 620 ml Diäthyläther versetzt man unter Rühren nacheinander mit 98,1 g Trimethylchlorsilan und 91,8 g Triäthylamin, wobei sich ein dicker Niederschlag bildet und die Temperatur auf 15° ansteigt. Man rührt 1 Stunde bei Raumtemperatur und lässt anschliessend 19 Stunden lang stehen. Dann wird unter Ausschluss von Feuchtigkeit genutscht, und das abgenutschte Material wird mit 200 ml Aether gewaschen. Das Filtrat wird unter Rühren und Kühlen im Eisbad innert 30 Minuten mit 430 ml einer 2,2M-Aethylmagnesiumbromid/Aether-Lösung versetzt, wobei es zu Gasentwicklung kommt und die Reaktionstemperatur auf 15° ansteigt. Das Reaktionsgemisch wird während 18 Stunden bei Raumtemperatur gerührt und dann langsam auf 350 ml einer gerührten, im Eisbad gekühlten 14-proz. wässrigen Ammoniumchloridlösung gegossen, wobei der pH des Hydrolysegemisches durch gleichzeitige Zugabe von 3N-Salzsäure zwischen 7 und 8 gehalten wird. Die organische Phase wird abgetrennt und mit gesättigter Natriumchloridlösung gewaschen. Die Wasserphasen werden mit insgesamt 1 l Methylenchlorid extrahiert. Die vereinigten organischen Phasen werden über Natriumsulfat getrocknet und im Vakuum eingedampft. Man nimmt das verbleibende Oel in 800 ml Aethanol auf und erhitzt die Lösung während 2 Stunden unter Rückfluss zum Sieden. Das

Lösungsmittel wird im Vakuum vollständig entfernt, und das Rohprodukt wird aus Aethylacetat/Hexan kristallisiert. Man erhält weisse Kristalle vom Schmelzpunkt 85-88°. Durch dreimaliges Umkristallisieren aus Aethylacetat/Hexan erhält man (S)-4-[(R)-2,2-Dimethyl-1,3-dioxolan-4-yl]-2-azetidinon vom Schmelzpunkt 92-93°; $[\alpha]_D^{20}$ = +13,6° (c=1, Aethylacetat).

d) Zu einem auf 15° vorgekühlten Gemisch aus 25,7 g (S)-4-[(R)-2,2-Dimethyl-1,3-dioxolan-4-yl]-2-azetidinon, 0,6 g Benzyltriäthylammoniumchlorid und 9,3 g pulverisiertem Kaliumhydroxyd in 150 ml Tetrahydrofuran tropft man unter starkem Rühren innert 10 Minuten eine Lösung von 19,1 g Allylbromid in 30 ml Tetrahydrofuran, wobei die Temperatur bei 15-20° gehalten wird. Man rührt noch 30 Minuten, verdünnt dann mit 200 ml Aethylacetat und wäscht das Reaktionsgemisch zweimal mit je 80 ml gesättigter Natriumchloridlösung. Die Wasserphasen werden noch mit 100 ml Aethylacetat nachextrahiert. Die organischen Phasen werden über Natriumsulfat getrocknet und im Vakuum eingedampft, worauf man das verbleibende Oel im Hochvakuum destilliert. Man erhält (S)-1-Allyl-4-[(R)-2,2-dimethyl--1,3-dioxolan-4-yl]-2-azetidinon als farbloses Oel vom Siedepunkt 82-87° (0,1 mm); $[\alpha]_D^{20}$ = +58,6° (c=1, Aethylacetat).

e) Zu einer Lösung von 21,1 g (S)-1-Allyl-4-[(R)-2,2-dimethyl-1,3-dioxolan-4-yl]-2-azetidinon in 180 ml Tetrahydrofuran werden 50 ml Wasser und 3 g p-Toluolsulfonsäure-Monohydrat zugegeben, und das Gemisch wird während 2,5 Stunden bei 65° gerührt. Die homogene Reaktionslösung wird abgekühlt, mit gesättigter Natriumcarbonatlösung neutralisiert und dann im Vakuum vollständig eingedampft. Der Rückstand wird mit insgesamt 1,4 l Methylenchlorid extrahiert; die Extrakte werden über Natriumsulfat getrocknet und vollständig eingedampft. Man erhält rohes (S)-1-Allyl-4-[(R)-1,2--dihydroxyäthyl]-2-azetidinon als farbloses Oel, das ohne

weitere Reinigung in die nächste Stufe eingesetzt wird.

f) Man versetzt eine Lösung von 15,0 g (S)-1-Allyl-4--[(R)-1,2-dihydroxyäthyl]-2-azetidinon in 50 ml Pyridin mit 26,0 g Triphenylchlormethan, rührt das Reaktionsgemisch während 1 Stunde und lässt dann während 64 Stunden bei Raumtemperatur stehen, wobei sich ein Niederschlag bildet. Das Lösungsmittel wird im Vakuum weitgehend entfernt, und der feste Rückstand wird zwischen 500 ml Aethylacetat und 200 ml kalter 3N-Salzsäure verteilt. Die organische Phase wird nacheinander mit je 100 ml Wasser, 100 ml 5-proz. Natriumbicarbonatlösung und 100 ml gesättigter Natriumchloridlösung gewaschen, über Natriumsulfat getrocknet und im Vakuum vom Lösungsmittel befreit. Umkristallisieren des festen Rückstandes aus Methylenchlorid/Hexan ergibt (S)-1--Allyl-4-[(R)-1-hydroxy-2-(triphenylmethoxy)äthyl]-2-azetidinon als weisse Kristalle vom Schmelzpunkt 125-128°.

g) Zu einer Lösung von 11,75 g (S)-1-Allyl-4-[(R)-1-hydroxy-2-(triphenylmethoxy)äthyl]-2-azetidinon in 80 ml Dimethylformamid und 6,4 ml Methyljodid gibt man portionenweise 0,76 g Natriumhydrid, wobei die Reaktionstemperatur durch Kühlen bei 20° gehalten wird. Man rührt während 28 Stunden bei Raumtemperatur, verdünnt das Reaktionsgemisch dann mit 300 ml Aethylacetat und wäscht viermal mit je 100 ml halbgesättigter Natriumchloridlösung. Die Wasserphasen werden mit 150 ml Aethylacetat nachextrahiert. Die organischen Phasen werden über Natriumsulfat getrocknet und im Vakuum vollständig eingedampft. Durch Kristallisieren des Rückstandes aus Aethylacetat/Hexan erhält man (S)-1--Allyl-4-[(R)-1-methoxy-2-(triphenylmethoxy)äthyl]-2-azetidinon als weisse Kristalle vom Schmelzpunkt 105-106°; $[\alpha]_D^{20}$ = +9,3° (c=1, Aethylacetat).

h) Zu einer Lösung von 0,84 g Kalium-tert.-butylat in 30 ml Dimethylformamid gibt man eine Lösung von 22,7 g (S)-1-Allyl-4-[(R)-1-methoxy-2-(triphenylmethoxy)äthyl]-2--

azetidinon, wobei die Temperatur durch Kühlen im Eisbad auf 23° gehalten wird. Das dunkel gefärbte Reaktionsgemisch wird 10 Minuten bei dieser Temperatur gerührt und dann auf 100 ml eiskalte 0,1M-Phosphatpufferlösung vom pH 7 gegossen. Man extrahiert mit insgesamt 400 ml Aethylacetat, wäscht die organischen Phasen mit gesättigter Natriumchloridlösung, trocknet sie über Natriumsulfat und dampft im Vakuum vollständig ein. Durch Chromatographieren des Rohproduktes an Kieselgel mit Aethylacetat/Hexan (1:3, v/v) erhält man (S)-4-[(R)-1-Methoxy-2-(triphenylmethoxy)-äthyl]-1-[(E/Z)-propenyl]-2-azetidinon (E:Z ca. 1:4) als gelbes Oel.

i) Zu einer Lösung von 9,1 g (S)-4-[(R)-1-Methoxy-2-(triphenylmethoxy)äthyl]-1-[(E/Z)-propenyl]-2-azetidinon (E/Z ca. 1:4) in einem Gemisch aus 50 ml Dimethoxyäthan, 32 ml Aceton, 25 ml Wasser und 8 ml Pyridin werden 6,7 g festes Kaliumpermanganat zugegeben und das Reaktionsgemisch wird während 1 Stunde gerührt. Dabei steigt die Reaktionstemperatur zunächst bis 45° und sinkt dann langsam wieder auf Raumtemperatur. Man nutscht dann vom Niederschlag ab, wäscht mit Aceton nach und verteilt das Filtrat zwischen Aethylacetat und Wasser. Die organische Phase wird über Natriumsulfat getrocknet und im Vakuum vom Lösungsmittel befreit, worauf man den Rückstand an 200 g Kieselgel mit Aethylacetat/Hexan (2:2, v/v) chromatographiert. Man erhält (S)-4-[(R)-1-Methoxy-2-(triphenylmethoxy)äthyl]-2-azetidinon als farbloses Oel.

j) Eine auf 0° gekühlte Lösung von 7,3 g (S)-4-[(R)-1-Methoxy-2-(triphenylmethoxy)äthyl]-2-azetidinon und 3,5 g tert.-Butyldimethylchlorsilan in 40 ml Dimethylformamid wird mit 2,3 g Triäthylamin versetzt, wobei sofort ein Niederschlag entsteht. Man rührt während 1,5 Stunden bei 0°, verdünnt dann das Reaktionsgemisch mit 150 ml Aethylacetat und extrahiert dreimal mit je 70 ml Wasser. Die Wasserphasen werden noch mit 100 ml Aethylacetat nachextrahiert; die

vereinigten organischen Phasen werden über Natriumsulfat getrocknet und im Vakuum vom Lösungsmittel befreit. Der Rückstand wird an 150 g Kieselgel mit Aethylacetat/Hexan (1:2, v/v) chromatographiert, und das so gereinigte Produkt wird aus Hexan kristallisiert. Man erhält (S)-1-(tert.-Butyldimethylsilyl)-4-[(R)-1-methoxy-2-(triphenylmethoxy)-äthyl]-2-azetidinon als weisse Kristalle vom Schmelzpunkt 119-121°.

k) Zu einer auf -60° vorgekühlten Lösung von 1,71 g Diisopropylamin in 50 ml Tetrahydrofuran tropft man innert 2 Minuten 9,6 ml einer 1,75M-Lösung von Butyllithium in Hexan. Man rührt während 13 Minuten bei -60°, kühlt dann auf -78° ab und tropft innert 4 Minuten eine Lösung von 7,68 g (S)-1-(tert.-Butyldimethylsilyl)-4-[(R)-1-methoxy-2-(triphenylmethoxy)äthyl]-2-azetidinon in 15 ml Tetrahydrofuran. Man rührt während 45 Minuten bei -78° und kühlt die Reaktionslösung dann auf -107° ab. Nun werden innert 2 Minuten eine Lösung von 2,23 g Acetaldehyd in 10 ml Tetrahydrofuran zugetropft, wobei die Temperatur vorübergehend auf -100° ansteigt. Man rührt noch 15 Minuten bei -105°, wärmt dann innert 5 Minuten auf -60° auf, versetzt die Reaktionslösung mit 30 ml einer 14-proz. wässrigen Ammoniumchloridlösung, verdünnt mit 150 ml Aethylacetat und wäscht das Reaktionsgemisch dreimal mit je 70 ml gesättigter Natriumchloridlösung. Die Wasserphasen werden mit 150 ml Aethylacetat nachextrahiert; die vereinigten organischen Phasen werden über Natriumsulfat getrocknet und im Vakuum vollständig eingedampft. Der Rückstand wird in 50 ml Methanol aufgenommen; die Lösung wird mit 1,5 g Ammoniumfluorid versetzt und während 1 Stunde bei Raumtemperatur gerührt. Man verdünnt mit Aethylacetat, dampft im Vakuum vollständig ein, nimmt den Rückstand in Aethylacetat auf und filtriert vom Ungelösten ab. Das Filtrat wird im Vakuum vom Lösungsmittel befreit, und der kristalline Rückstand wird zweimal aus Aethylacetat/Hexan umkristallisiert. Man erhält (3S,4S)-3-[(R)-1-Hydroxyäthyl]-4-[(R)-1-methoxy-2-

(triphenylmethoxy)äthyl]-2-azetidinon als weisse Kristalle vom Schmelzpunkt 161-163°.

l) Eine auf 0° vorgekühlte Lösung von 1,94 g (3S,4S)-3--[(R)-1-Hydroxyäthyl]-4-[(R)-1-methoxy-2-(triphenylmethoxy)-äthyl]-2-azetidinon und 1,01 g 4-Dimethylaminopyridin in 4,5 ml Methylenchlorid wird mit einer Lösung von 1,07 g Chlorameisensäure-p-nitrobenzylester in 3 ml Methylenchlorid versetzt. Die Reaktionslösung wird während 2 Stunden bei 0° gerührt, wobei sich ein Niederschlag bildet. Das Reaktionsgemisch wird dann mit 50 ml Methylenchlorid verdünnt und nacheinander mit 40 ml 0,3N-Salzsäure, 40 ml Wasser, 40 ml 5-proz. Natriumhydrogencarbonat- und 40 ml gesättigter Natriumchloridlösung gewaschen. Die organische Phase wird über Natriumsulfat getrocknet und im Vakuum vom Lösungsmittel befreit. Das Rohprodukt wird durch Chromatographieren an Kieselgel mit Aethylacetat/Hexan (1:1, v/v) als Eluens gereinigt. Man erhält (3S,4S)-4-[(R)-1-Methoxy--2-(triphenylmethoxy)äthyl]-3-[(R)-1-[(p-nitrobenzyloxycarbonyl)oxy]äthyl]-2-azetidinon als amorphen Schaum mit einem Rf-Wert von 0,39; Kieselgel, Aethylacetat/Hexan (2:1).

m) Eine Lösung von 2,0 g (3S,4S)-4-[(R)-1-Methoxy-2-triphenylmethoxy)äthyl]-3-[(R)-1-[[(p-nitrobenzyloxycarbonyl)-oxy]äthyl]-2-azetidinon, 0,33 g Glyoxylsäure-Monohydrat und 0,51 g N-Aethyldiisopropylamin in 5 ml Dimethylformamid wird während 20 Stunden bei Raumtemperatur stehen gelassen, dann mit 0,85 g p-Nitrobenzylbromid versetzt und weitere 20 Stunden bei Raumtemperatur stehen gelassen. Die Reaktionslösung wird im Vakuum weitgehend eingedampft, und das verbleibende Oel wird in 40 ml Aethylacetat aufgenommen. Die Lösung wird mit gesättigter Natriumhydrogencarbonatlösung und gesättigter Natriumchloridlösung gewaschen. Die organische Phase wird über Natriumsulfat getrocknet und im Vakuum vom Lösungsmittel befreit. Der Rückstand wird an Kieselgel mit Aethylacetat/Methylenchlorid (1:4, v/v) als Elutionsmittel chromatographiert. Man erhält 2-[(2S,3S)-2--

[(R)-1-Methoxy-2-(triphenylmethoxy)äthyl]-3-[(R)-1-[(p-nitrobenzyloxycarbonyl)oxy]äthyl]-4-oxo-1-azetidinyl]-2-hydroxy-essigsäure-p-nitrobenzylester (Diastereomerengemisch) als amorphen Schaum mit einem Rf-Wert von 0,70; Kieselgel, Aethylacetat/Methylenchlorid (1:3).

n) Eine auf -30° vorgekühlte Lösung von 1,85 g 2-[(2S,-3S)-2-[(R)-1-Methoxy-2-(triphenylmethoxy)äthyl]-3-[(R)-1-[(p-nitrobenzyloxycarbonyl)oxy]äthyl]-4-oxo-1-azetidinyl]-2-hydroxyessigsäure-p-nitrobenzylester in 19 ml Tetrahydrofuran wird nacheinander mit 0,33 g 2,6-Lutidin und dann innert 1 Minute mit 0,35 g Thionylchlorid in 4 ml Tetrahydrofuran versetzt. Das Reaktionsgemisch wird 20 Minuten bei -20° und weitere 20 Minuten bei Raumtemperatur gerührt. Dann wird das Reaktionsgemisch mit Toluol verdünnt und durch Nutschen vom Niederschlag befreit. Das Filtrat wird im Vakuum vollständig eingedampft. Der Rückstand wird in 9 ml Dimethylformamid gelöst; die Lösung wird mit 0,75 g Triphenylphosphin versetzt und während 1 Stunde bei Raumtemperatur gerührt. Dann wird das Lösungsmittel im Vakuum weitgehend entfernt, und das verbleibende Oel wird zwischen Methylenchlorid und 0,2M-Phosphatpuffer vom pH 7 verteilt. Die organischen Phasen werden über Natriumsulfat getrocknet, worauf man das Lösungsmittel im Vakuum abdampft und den Rückstand an Kieselgel chromatographiert. Man verwendet nacheinander Methylenchlorid, Aethylacetat/Hexan (1:2, v/v) und Aethylacetat als Elutionsmittel. Man erhält 2-[(2S,3S)-2--[(R)-1-Methoxy-2-(triphenylmethoxy)äthyl]-3-[(R)-1-[(p-nitrobenzyloxycarbonyl)oxy]äthyl]-4-oxo-azetidin-1-yl]-2-(triphenylphosphoranyliden)essigsäure-p-nitrobenzylester als amorphen Schaum mit einem Rf-Wert von 0,22; Kieselgel, Aethylacetat/Hexan (1:1).

o) Eine Lösung von 0,58 g 2-[(2S,3S)-2-[(R)-1-Methoxy-2--(triphenylmethoxy)äthyl]-3-[(R)-1-[(p-nitrobenzyloxycarbonyl)oxy]äthyl]-4-oxo-azetidin-1-yl]-2-(triphenylphosphoranyliden)essigsäure-p-nitrobenzylester in 8 ml 80-proz. wässriger

Essigsäure wird während 1,5 Stunden auf 60° erhitzt. Das Reaktionsgemisch wird abgekühlt, mit Aethylacetat verdünnt und im Vakuum weitgehend vom Lösungsmittel befreit. Man nimmt den Rückstand in Aethylacetat auf, wäscht die Lösung mit 5-proz. Natriumhydrogencarbonat- und gesättigter Natriumchloridlösung, trocknet über Natriumsulfat und dampft im Vakuum vollständig ein. Der Rückstand wird an Kieselgel mit Aethylacetat/Hexan (2:1, v/v) als Elutionsmittel chromatographiert. Man erhält 2-[(2S,3S)-2-[(R)-2--Hydroxy-1-methoxyäthyl]-3-[(R)-1-[(p-nitrobenzyloxycarbonyl)-oxy]äthyl]-4-oxo-azetidin-1-yl]-2-(triphenylphosphoranyliden) essigsäure-p-nitrobenzylester als amorphen Schaum mit einem Rf-Wert von 0,30; Kieselgel, Aethylacetat.

p) 80 mg 2-[(2S,3S)-2-[(R)-2-Hydroxy-1-methoxyäthyl]-3--[(R)-1-[(p-nitrobenzyloxycarbonyl)oxy]äthyl]-4-oxo-azetidin-1-yl]-2-(triphenylphosphoranyliden)essigsäure-p-nitrobenzylester werden in 8 ml Methylenchlorid gelöst, worauf man mit einer fein pulverisierten Mischung aus 100 mg Pyridiniumdichromat, 50 mg Magnesiumsulfat, 50 mg Molekularsieb (4A) versetzt, das Reaktionsgemisch während 40 Minuten bei Raumtemperatur rührt und dann durch ein Filter aus Kieselgel nutscht und mit Aethylacetat nachwäscht. Das Filtrat wird im Vakuum konzentriert und an 5 g Kieselgel mit Aethylacetat/Hexan (1:1, v/v) chromatographiert. Man erhält (4S,5S,-6S)-4-Methoxy-6-[(R)-1-[(p-nitrobenzyloxycarbonyl)oxy]äthyl]-7-oxo-1-azabicyclo[3.2.0]hept-2-en-2-carbonsäure-p-nitrobenzylester als amorphen Schaum.

q) Eine Lösung von 20 mg (4S,5S,6S)-4-Methoxy-6-[(R)-1--[(p-nitrobenzyloxycarbonyl)oxy]äthyl]-7-oxo-1-azabicyclo-[3.2.0]hept-2-en-2-carbonsäure-p-nitrobenzylester in 4 ml Aethylacetat wird mit einer Lösung von 3 mg Natriumhydrogencarbonat in 4 ml 0,012M-Phosphatpufferlösung vom pH 7 versetzt. Das Gemisch wird in Gegenwart von 35 mg Palladium/Kohle (10%) während 15 Minuten bei Normaldruck hydriert. Man filtriert dann vom Katalysator ab, verdünnt mit

Wasser und extrahiert das Filtrat mit drei Portionen Aethylacetat. Aus der wässrigen Phase erhält man (4S,5S,-6S)-6-[(R)-1-Hydroxyäthyl]-4-methoxy-7-oxo-1-azabicyclo-[3.2.0]hept-2-en-2-carbonsäure-Natriumsalz; UV ($H_2O$): $\lambda_{max}$ = 265 nm (mit Hydroxylamin auslöschbar).

Beispiel 2

a) Eine Lösung von 151 mg Azodicarbonsäurediisopropylester in 1 ml Tetrahydrofuran wird innert 1 Stunde zu einer Lösung von 206 mg (S)-1-Allyl-4-[(R)-1-hydroxy-2-(triphenylmethoxy)äthyl]-2-azetidinon, 196 mg Triphenylphosphin und 73 mg Benzoesäure in 1 ml Tetrahydrofuran zugetropft, und die Reaktionslösung wird während 20 Stunden bei Raumtemperatur stehen gelassen. Das Lösungsmittel wird im Vakuum entfernt, und der Rückstand wird an Kieselgel mit Aethylacetat/Hexan/Methylenchlorid (1:2:2, v/v/v) als Elutionsmittel chromatographiert. Man erhält (S)-1-Allyl-4--[(S)-1-benzoyloxy-2-(triphenylmethoxy)äthyl]-2-azetidinon als farbloses Oel mit einem Rf-Wert von 0,62; Kieselgel, Methylenchlorid/Aethylacetat/Hexan (2:1:1).

b) Eine Lösung von 150 mg (S)-1-Allyl-4-[(S)-1-benzoyloxy-2-(triphenylmethoxy)äthyl]-2-azetidinon in 10 ml 0,05M-methanolischer Kaliumhydroxylösung wird während 3 Stunden bei Raumtemeratur stehen gelassen. Die Reaktionslösung wird mit Aethylacetat verdünnt und mit gesättigter Natriumchloridlösung gewaschen. Die organische Phase wird über Natriumsulfat getrocknet und im Vakuum vom Lösungsmittel befreit. Der Rückstand wird an Kieselgel mit Methylenchlorid/Aethylacetat/Hexan (2:1:1, v/v/v) als Elutionsmittel chromatographiert. Man erhält nach Umkristallisation aus Aethylacetat/Hexan (S)-1-Allyl-4-[(S)-1-hydroxy-2-(triphenylmethoxy)äthyl]-2-azetidinon als weisse Kristalle vom Schmelzpunkt 135-137°.

c) Aus (S)-1-Allyl-4-[(S)-1-hydroxy-2-(triphenylmethoxy)-äthyl]-2-azetidinon erhält man in Analogie zu den Beispielen 1(g) bis 1(p) (4R,5S,6S)-4-Methoxy-6-[(R)-1-[(p-nitrobenzyloxycarbonyl)oxy)äthyl]-7-oxo-1-azabicyclo[3.2.0]hept-2-en-2-carbonsäure-p-nitrobenzylester.

d) (4R,5S,6S)-4-Methoxy-6-[(R)-1-[(p-nitrobenzyloxycarbonyl)oxy]äthyl]-7-oxo-1-azabicyclo[3.2.0]hept-2-en-2-carbonsäure--p-nitrobenzylester wird in Analogie zu Beispiel 1(q) hydriert. Man erhält (4R,5S,6S)-6-[(R)-1-Hydroxyäthyl]-4-methoxy-7-oxo-1-azabicyclo[3.2.0]hept-2-en-2-carbonsäure--Natriumsalz; UV ($H_2O$): $\lambda_{max}$ = 266 nm (mit Hydroxylamin auslöschbar).

## Beispiel 3

a) Eine Lösung von 15,5 g (3S,4S)-1-Methoxy-2-(triphenylmethoxy)äthyl]-3-[(R) -1-[(p-nitrobenzyloxycarbonyl)oxy]-äthyl]-2-azetidinon in 200 ml 80-proz. wässriger Essigsäure wird während 1 Stunde auf 65° erhitzt. Das Reaktionsgemisch wird abgekühlt, mit Aethylacetat verdünnt und im Vakuum weitgehend vom Lösungsmittel befreit. Man nimmt den Rückstand in Aethylacetat auf, wäscht die Lösung mit gesättigter Natriumcarbonat- und gesättigter Natriumchloridlösung, trocknet sie über Natriumsulfat und dampft sie im Vakuum ein. Der Rückstand wird an Kieselgel mit Aethylacetat als Elutionsmittel chromatographiert. Man erhält nach Kristallisation aus Aethylacetat/Hexan (3S,4S)-4-[(R)-2-Hydroxy-1--methoxyäthyl]-3-[(R) -1-[(p-nitrobenzyloxycarbonyl)oxy]-äthyl]-2-azetidinon als weisse Kristalle vom Schmelzpunkt 84-86°.

b) Eine Lösung von 1,08 g (3S,4S)-4-[(R)-2-Hydroxy-1--methoxyäthyl]-3-[(R) -1-[(p-nitrobenzyloxycarbonyl)oxy]-äthyl]-2-azetidinon in 3 ml Dioxan wird mit 18 ml 0,2M--Phosphatpuffer vom pH 7 versetzt, worauf man den pH des Gemisches mit 10-proz. Phosphorsäure auf 5,0 einstellt. Man

gibt 0,72 g Kaliumpermanganat dazu und rührt das Reaktionsgemisch während 5 Stunden bei Raumtemperatur, wobei der pH durch Zugabe von 10-proz. Phosphorsäure bei 5,0 gehalten wird. Dann wird das Gemisch unter Rühren im Eisbad mit 10-proz. Schwefelsäure auf pH 3 angesäuert, und das überschüssige Kaliumpermanganat und das Mangandioxid werden mit gesättigter Natriumhydrogensulfitlösung reduziert. Die klare Lösung wird durch Zugabe von Natronlauge wieder auf pH 8 gestellt und dann mit Aethylacetat extrahiert. Die Wasserphase wird mit 10-proz. Schwefelsäure auf pH 3 gestellt, mit Ammoniumsulfat gesättigt und mit Aethylacetat extrahiert. Die organischen Phasen werden über Natriumsulfat getrocknet und im Vakuum eingedampft. Durch Kristallisation des Rückstandes aus Aethylacetat/Hexan erhält man (αR,2S,3S)-α-Methoxy-3-[(R)-1 -[(p-nitrobenzyloxycarbonyl)oxy]äthyl] -4-oxo-3-azetidin-essigsäure als weisse Kristalle vom Schmelzpunkt 144-145°.

c) Eine Lösung von 0,92 g (αR,2S,3S)-α-Methoxy-3-[(R)-1-[(p-nitrobenzyloxycarbonyl)oxy]äthyl]-4-oxo-3-azetidin-essigsäure in 12 ml Tetrahydrofuran wird mit 0,39 g 1,1'-Carbonyldiimidazol versetzt und anschliessend während 3,5 Stunden bei Raumtemperatur gerührt. Dann werden der Reaktionslösung 1,36 g wasserfreies Malonsäure-mono-p-nitrobenzylester-Magnesiumsalz zugegeben, und das Gemisch wird während 18 Stunden bei Raumtemperatur gerührt. Das Reaktionsgemisch wird mit Aethylacetat verdünnt und nacheinander mit 0,2M-Phosphatpuffer vom pH 3, Wasser, gesättigter Natriumhydrogencarbonat- und gesättigter Natriumchloridlösung gewaschen. Die organische Phase wird über Natriumsulfat getrocknet und im Vakuum eingedampft. Das Rohprodukt wird an Kieselgel mit Aethylacetat/Hexan (3:1, v/v) als Elutionsmittel chromatographiert. Man erhält (3S,4S)-4-[(R)-1-Methoxy-3-(p-nitrobenzyloxycarbonyl)-2-oxopropyl]-3-[(R)-1-[(p-nitrobenzyloxycarbonyl)oxy]äthyl]-2-azetidinon (Keto/Enol-Gemisch) als amorphen Schaum.

d) Zu einer Lösung von 560 mg (3S,4S)-4-[(R)-1-Methoxy-3--(p-nitrobenzyloxycarbonyl)-2-oxopropyl]-3-[(R)-1 -[(p--nitrobenzyloxycarbonyl)oxy]äthyl]-2-azetidinon und 227 mg Tosylazid in 3 ml Acetonitril gibt man bei 0° 180 mg Triäthylamin. Das Reaktionsgemisch wird während 4 Stunden bei Raumtemperatur gerührt, dann mit Aethylacetat verdünnt und nacheinander mit 5-proz. Phosphorsäure, Wasser, gesättigter Natriumhydrogencarbonat- und gesättigter Natriumchloridlösung gewaschen. Die organische Phase wird über Natriumsulfat getrocknet und im Vakuum eingedampft. Das Rohprodukt wird an Kieselgel mit Aethylacetat/Methylenchlorid/Hexan (2:1:1, v/v/v) als Elutionsmittel chromatographiert. Man erhält (3S,4S)-4-[(R)-3-Diazo-1-methoxy-3-(p-nitrobenzyloxycarbonyl) -2-oxopropyl]-3-[(R)-1-[(p-nitrobenzyloxycarbonyl)oxy]äthyl] -2-azetidinon als amorphen Schaum; IR-Spektrum ($CHCl_3$): Banden u.a. bei 3414, 2151, 1771 (Schulter bei 1745), 1718 und 1658 $cm^{-1}$.

e) Eine Lösung von 350 mg (3S,4S)-4-[(R)-3-Diazo-1--methoxy-3-(p-nitrobenzyloxycarbonyl) -2-oxopropyl]-3--[(R)-1-[(p-nitrobenzyloxycarbonyl)oxy]äthyl]-2-azetidinon in 10 ml Toluol wird mit 5 mg Rhodium(II)acetat versetzt und während 1 Stunde bei 85° gerührt. Das Reaktionsgemisch wird abgekühlt, mit Aethylacetat verdünnt, filtriert und im Vakuum eingedampft. Der Rückstand wird an silanisiertem Kieselgel mit Aethylacetat/Hexan (1:1, v/v), als Elutionsmittel chromatographiert. Man erhält (4R,5S,-6S)-3-Methoxy-6-[(R)-1-[(p-nitrobenzyloxycarbonyl)oxy]-äthyl]-3,7-dioxo-1-azabicyclo[3.2.0]heptan -2-carbonsäure--p-nitrobenzylester als farbloses Oel. IR-Spektrum ($CHCl_3$): Banden u.a. bei 1775 und 1750 $cm^{-1}$.

f) Eine Lösung von 270 mg (4R,5S,6S)-3-Methoxy-6-[(R)-1 -[(p-nitrobenzyloxycarbonyl)oxy]äthyl]-3,7-dioxo-1-azabicyclo[3.2.0]heptan -2-carbonsäure-p-nitrobenzylester in 6 ml Acetonitril wird auf 0° abgekühlt und nacheinander mit 153 mg Phosphorsäure-diphenylesterchlorid und 71 mg Diisopropylamin versetzt, wobei sich ein Niederschlag bildet. Das Reaktionsgemisch wird 20 Minuten bei 0° gerührt und dann mit einer Lösung von 168 mg (S)-3-Mercapto-1-(p-nitrobenzyloxycarbonyl)-pyrrolidin und 71 mg Diisopropylamin in 0,5 ml Acetonitril versetzt. Das Reaktionsgemisch wird 1 Stunde bei 0° gerührt, dann mit Aethylacetat verdünnt und mit Wasser, 5-proz. Natriumhydrogencarbonat- und gesättigter Natriumchloridlösung gewaschen. Die organische Phase wird über Natriumsulfat getrocknet und im Vakuum vom Lösungsmittel befreit. Der Rückstand wird an Kieselgel mit Aethylacetat/Methylenchlorid/Hexan (2:1:1, v/v/v) chromatographiert. Man erhält (4R,5S,6S)-4-Methoxy-6-[(R)-1-[(p--nitrobenzyloxycarbonyl)oxy]äthyl]-3-[(S) -1-(p-nitrobenzyloxycarbonyl) -3-pyrrolidinyl]thio]-7-oxo-1-azabicyclo[3.2.0]hept-2-en-2-carbonsäure -p-nitrobenzylester als amorphen Schaum.

g) Ein Gemisch von 168 mg (4R,5S,6S)-4-Methoxy-6-[(R)-1-[(p-nitrobenzyloxycarbonyl)oxy]äthyl] -3-[(S)-1-(p-nitrobenzyloxycarbonyl) -3-pyrrolidinyl]thio]-7-oxo-1-azabicyclo[3.2.0]hept-2-en -2-carbonsäure-p-nitrobenzylester in 10 ml Tetrahydrofuran und 10 ml 0,1M-Phosphatpuffer vom pH 7 wird in Gegenwart von 200 mg Palladium/Kohle (10%) während 1,5 Stunden bei Normaldruck hydriert. Man filtriert vom Katalysator ab, verdünnt das Filtrat mit Wasser und extrahiert es mit Aethylacetat. Aus der wässrigen Phase erhält man (4R,5S,6S)-6-[(R)-1-Hydroxyäthyl]-4-methoxy--3-[(S) -3-pyrrolidinylthio]-7-oxo-1-azabicyclo[3.2.0]-hept -2-en-2-carbonsäure; UV ($H_2O$): $\lambda_{max}$ = 299 nm (Verschiebung von $\lambda_{max}$ nach 268 nm mit Hydroxylamin).

Beispiel 4

Eine gemäss Beispiel 3g) erhaltene wässrige Lösung von (4R,5S,6S)-6-[(R)-1-Hydroxyäthyl]-4-methoxy-3-[(S) -3-pyrrolidinylthio]-7-oxo-1-azabicyclo[3.2.0]hept -2-en-2--carbonsäure wird im Vakuum auf 5 ml konzentriert und bei

0° durch Zugabe von 1N-Natronlauge auf pH 8,5 gestellt. Das Reaktionsgemisch wird innert 10 Minuten portionenweise mit 75 mg Aethyl-acetimidat-hydrochlorid versetzt, wobei der pH durch Zugabe von 0,1N-Natronlauge bei 8,5 gehalten wird. Man rührt noch 30 Minuten bei 0°, neutralisiert dann das Reaktionsgemisch durch Zugabe von 0,5N-Salzsäure und engt im Vakuum auf ca. 3 ml ein. Die konzentrierte Lösung wird an MCI-Gel CHP20P (Mitsubishi Chemical Industries, Ltd.) mit 2-proz. wässrigem Aceton chromatographiert. Nach Lyophilisieren erhält man (4R,5S,6S)-6-[(R)-1-Hydroxyäthyl]-3--[[(S)-1-(1-iminoäthyl) -3-pyrrolidinyl]thio]-4-methoxy--7-oxo-1-azabicyclo[3.2.0]hept-2-en -2-carbonsäure als weisses, amorphes Pulver; UV ($H_2O$): $\lambda_{max}$ = 299 nm (Verschiebung von $\lambda_{max}$ nach 269 nm mit Hydroxylamin).

Beispiel 5

a) Zu einer Lösung von 60,3 g (S)-1-Allyl-4-[(R)-1--hydroxy-2-(triphenylmethoxy)äthyl] -2-azetidinon in 350 ml Dimethylformamid und 47 ml Aethyljodid gibt man portionenweise 22 g Natriumhydrid-Dispersion (55% in Oel). Das Reaktionsgemisch wird während 24 Stunden unter Argon auf 50° erhitzt. Es wird nochmals 1 g Natriumhydrid-Dispersion zugegeben und weitere 24 Stunden auf 50° erhitzt. Das Gemisch wird auf Raumtemperatur gekühlt und unter Rühren zunächst mit 100 ml Aethanol und dann mit 100 ml Wasser versetzt. Ueberschüssiges Aethyljodid und Aethanol werden im Vakuum entfernt, und die verbleibende Reaktionslösung wird zwischen Aethylacetat und verdünnter Natriumchloridlösung verteilt. Die organische Phase wird über Natriumsulfat getrocknet und im Vakuum eingedampft. Der Rückstand wird aus Aethylacetat/Hexan umkristallisiert. Man erhält (S)-4-[(R)-1-Aethoxy-2-(triphenylmethoxy)äthyl]-1-allyl -2-azetidinon als weisse Kristalle vom Schmelzpunkt 117-118°.

b) Aus (S)-4-[(R)-1-Aethoxy-2-(triphenylmethoxy)äthyl]-1--allyl -2-azetidinon erhält man in Analogie zu den Angaben in den Beispielen 1h) bis 1l) und 3a) bis 3e) den (4R,5S,-6S)-4-Aethoxy-6-[(R)-1 -[(p-nitrobenzyloxycarbonyl)oxy]-äthyl]-3,7-dioxo -1-azabicyclo[3.2.0]heptan-2-carbonsäure--p-nitrobenzylester als farbloses Oel.

c) Aus (4R,5S,6S)-3-Aethoxy-6-[(R)-1 -[(p-nitrobenzyloxycarbonyl)oxy]äthyl]-3,7-dioxo -1-azabicyclo[3.2.0]heptan-2--carbonsäure-p-nitrobenzylester erhält man in Analogie zu den Angaben in den Beispielen 3f) und 3g) und nach chromatographischer Reinigung an MCI-Gel CHP20P mit 2-proz. wässrigem Aceton als Elutionsmittel und Lyophilisieren die (4R,5S,6S)-4-Aethoxy-6-[(R)-1-hydroxyäthyl]-3-[(S)-3-pyrrolidinylthio]-7-oxo-1-azabicyclo[3.2.0]hept -2-en-2--carbonsäure als weisses, amorphes Pulver; UV ($H_2O$): $\lambda_{max}$ = 300 nm (Verschiebung von $\lambda_{max}$ nach 269 nm mit Hydroxylamin).

## Beispiel 6

Aus (4R,5S,6S)-4-Aethoxy-6-[(R)-1-hydroxyäthyl]-3-[(S)-3-pyrrolidinylthio]-7-oxo-1-azabicyclo[3.2.0]hept -2-en-2--carbonsäure erhält man in Analogie zu den Angaben in Beispiel 4 die (4R,5S,6S)-4-Aethoxy-6-[(R)-1-hydroxyäthyl]-3--[[(S)-1 -(1-iminoäthyl)-3-pyrrolidinyl]thio]-7-oxo -1-aza-bicyclo[3.2.0]hept-2-en-2-carbonsäure als weisses, amorphes Pulver; UV ($H_2O$): $\lambda_{max}$ = 300 nm (Verschiebung von $\lambda_{max}$ nach 270 nm mit Hydroxylamin).

## Beispiel 7

a) Eine Lösung von 171 mg (4R,5S,6S)-4-Aethoxy-6-[(R)-1 -[(p-nitrobenzyloxycarbonyl)oxy]äthyl]-3,7-dioxo -1-azabi-cyclo[3.2.0]heptan-2-carbonsäure-p-nitrobenzylester in 4 ml Acetonitril wird auf 0° abgekühlt und nacheinander mit 108 mg Phosphorsäure-diphenylesterchlorid und 64 mg Aethyldiisopropylamin versetzt. Das Reaktionsgemisch wird 20 Minuten bei 0° gerührt, wobei sich ein Niederschlag bildet, und dann wird eine Lösung von 45 mg 2-Mercaptopyrimidin und 64 mg Aethyldiisopropylamin in 0,3 ml Acetonitril zugegeben. Nach 2 Stunden Rühren bei Raumtemperatur wird das Reaktionsgemisch mit Aethylacetat verdünnt und nacheinander mit Wasser, gesättigter Natriumcarbonat- und gesättigter Natriumchloridlösung gewaschen. Die organische Phase wird über Natriumsulfat getrocknet und im Vakuum vom Lösungsmittel befreit. Der Rückstand wird an Kieselgel mit Methylenchlorid/Aethylacetat/Hexan (6:1:1, v/v/v) chromatographiert. Man erhält nach Umkristallisieren aus Methylenchlorid/Hexan (4R,5S,6S)-4-Aethoxy-6-[(R)-1 -[(p-nitrobenzyloxycarbonyl)oxy]äthyl]-3-(2-pyrimidinylthio) -7-oxo-1-azabicyclo[3.2.0]hept-2-en-2-carbonsäure -p-nitrobenzylester als blassgelbe Kristalle vom Schmelzpunkt 174-177°.

b) Der (4R,5S,6S)-4-Aethoxy-6-[(R)-1 -[(p-nitrobenzyloxycarbonyl)oxy]äthyl]-3-(2-pyrimidinylthio) -7-oxo-1-azabicyclo[3.2.0]hept-2-en-2-carbonsäure -p-nitrobenzylester wird in Analogie zu den Angaben in Beispiel 3g) hydriert. Aus der wässrigen Phase erhält man das (4R,5S,6S)-4-Aethoxy-6-[(R)-1-hydroxyäthyl]-3 -(2-pyrimidinylthio)-7-oxo-1-azabicyclo[3.2.0]hept-2-en -2-carbonsäure-Natriumsalz; UV ($H_2O$): $\lambda_{max}$ = 298 nm (Verschiebung von $\lambda_{max}$ nach 255 nm mit Hydroxylamin).

Beispiel 8

a) Eine Lösung von 0,5 g (S)-1-Allyl-4-[(R)-1-hydroxy-2--(triphenylmethoxy)äthyl] -2-azetidinon in 4 ml Dimethylformamid wird mit 3 g Isopentyljodid und 0,79 g Silber(I)-oxid versetzt. Das Reaktionsgemisch wird während 3,5 Stunden auf 80° erhitzt, dann auf Raumtemperatur abgekühlt, mit Methylenchlorid verdünnt und während 10 Minuten gerührt. Die Reaktionslösung wird vom Niederschlag abdekantiert und im Vakuum eingeengt, worauf man den Rückstand zwischen

Aethylacetat und Wasser verteilt. Die organische Phase wird über Natriumsulfat getrocknet und im Vakuum vom Lösungsmittel befreit. Das Rohprodukt wird an Kieselgel mit Aethylacetat/Hexan (2:1, v/v) chromatographiert. Man erhält (S)-1-Allyl-4-[(R)-1-isopentyloxy-2-(triphenylmethoxy)äthyl]-[(R)-1-[(p-nitrobenzyloxycarbonyl)oxy]äthyl]-2-azetidinon als farbloses Oel.

b) Aus (S)-1-Allyl-4-[(R)-1-isopentyloxy-2-(triphenylmethoxy)äthyl]-[(R)-1-[(p-nitrobenzyloxycarbonyl)oxy]äthyl]-2-azetidinon erhält man in Analogie zu den Angaben in den Beispielen 1h) bis 1l) und 3a) bis 3g) die (4R,5S,6S)-6--[(R)-1-hydroxyäthyl]-4-(1-isopentyloxy) -3-[(S)-3-pyrrolidinylthio]-7-oxo-1-azabicyclo[3.2.0]hept-2-en-2-carbonsäure; UV ($H_2O$): $\lambda_{max}$ = 300 nm (Verschiebung von $\lambda_{max}$ nach 272 nm mit Hydroxylamin).

Beispiel 9

a) Eine Lösung von 0,5 g (S)-1-allyl-4-[(R)-1-hydroxy-2--(triphenylmethoxy)äthyl] -2-azetidinon in 2,5 ml Dimethylformamid wird mit 0,057 g Natriumhydrid-Dispersion (ca. 55% in Oel) und 0,38 g Diphenyliodoniumchlorid versetzt. Das Reaktionsgemisch wird 2 Stunden bei Raumtemperatur und 2 Stunden bei 50° gerührt und dann zwischen Aethylacetat und Wasser verteilt. Die organische Phase wird über Natriumsulfat getrocknet und im Vakuum vom Lösungsmittel befreit. Der Rückstand wird an Kieselgel mit Aethylacetat/-Hexan (1:2, v/v) chromatographiert. Man erhält nach Kristallisieren aus Aethylacetat/Hexan (S)-1-Allyl-4-[(R)-1--phenoxy-2-(triphenylmethoxy)äthyl] -2-azetidinon als weisse Kristalle vom Schmelzpunkt 135-136°.

b) Aus (S)-1-Allyl-4-[(R)-1-phenoxy-2-(triphenylmethoxy)-äthyl] -2-azetidinon erhält man in Analogie zu den Angaben in den Beispielen 1h) bis 1l) und 3a) bis 3g) die (4R,5S,-6S)-6-[(R)-1-hydroxyäthyl]-4-phenoxy -3-[(S)-3-pyrroli-

0170019

dinylthio]-7-oxo-1-azabicyclo[3.2.0]hept -2-en-2-carbonsäure; UV ($H_2O$): $\lambda_{max}$ = 299 nm.

## Beispiel A

Die in den Beispielen 1 bis 9 beschriebenen erfindungsgemässen Endprodukte können wie folgt als Wirkstoffe zur Herstellung von Arzneimitteln verwendet werden:

a) Es wird in üblicher Weise eine Tablette folgender Zusammensetzung hergestellt:

| | |
|---|---|
| Wirkstoff | 250 mg |
| Lactose | 70 mg |
| Maisstärke | 65 mg |
| Polyvinylpyrrolidon | 10 mg |
| Magnesiumstearat | 5 mg |
| | 400 mg |

Mit dem Wirkstoff, der Lactose, dem Polyvinylpyrrolidon und 40 Gewichtsteilen der Maisstärke wird in üblicher Weise ein Granulat hergestellt. Dieses wird mit den restlichen 25 Gewichtsteilen Maisstärke und 5 Gewichtsteilen Magnesiumstearat vermischt und zu Tabletten gepresst.

b) Herstellung von Trockenampullen für die intramuskuläre Verabreichung:

Es wird in üblicher Weise ein Lyophilisat von 500 mg des Wirkstoffes oder gegebenenfalls dessen Natriumsalzes hergestellt und in eine Ampulle abgefüllt. Vor der Verabreichung wird das Lyophilisat mit 2,5 ml einer 2-proz. wässrigen Lidocainhydrochlorid-Lösung versetzt.

c) Es wird in üblicher Weise eine Gelatine-Steckkapsel folgender Zusammensetzung hergestellt:

| | |
|---|---|
| Wirkstoff | 500 mg |
| Wasserlösliches Polyvinylpyrrolidon | 20 mg |
| Mannit | 20 mg |
| Talk | 15 mg |
| Magnesiumstearat | 2 mg |
| | 557 mg |

## Patentansprüche

1. Carbapeneme der allgemeinen Formel

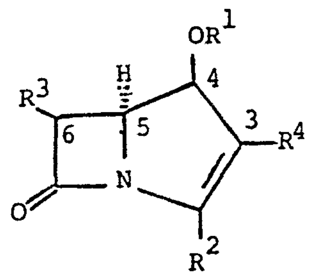

I

worin $R^1$ niederes Alkyl, Aryl oder Aryl-niederes Alkyl, $R^2$ Carboxy oder 5-Tetrazolyl, $R^3$ Wasserstoff oder einen für die 6-Stellung von Carbapenemen üblichen Substituenten und $R^4$ Wasserstoff oder einen für die 3-Stellung von Carbapenemen üblichen Substituenten bedeuten,

herkömmliche, unter physiologischen Bedingungen hydrolysierbare Ester von Verbindungen der Formel I, worin $R^2$ Carboxy bedeutet, und pharmazeutisch annehmbare Salze davon und Hydrate dieser Stoffe.

2. Verbindungen nach Anspruch 1, worin $R^3$ Wasserstoff, niederes Alkyl oder niederes Hydroxyalkyl, $R^4$ Wasserstoff oder die Gruppe $-S-(A)_m-(Q)_n-(A')_p-R^5$ (a), A und A' je niederes Alkylen oder niederes Alkenylen, m, n und p je die Zahl 0 oder 1, jedoch nicht alle gleichzeitig die Zahl 0, Q $(C_{3-6})$-Cycloalkylen, $(C_{3-6})$-Cycloalkenylen, Phenylen, 4- bis 7-gliedriges Heterocycloalkylen oder Heterocycloalkenylen oder 5- bis 6-gliedriges Heteroarylen und $R^5$ Wasserstoff, Halogen, Hydroxy, Nitro, Cyano, niederes Alkoxy, niederes Alkanoyl, niederes Alkanoyloxy, niederes Alkylthio, niederes Alkylsulfinyl, niederes Alkylsulfonyl, Carboxy, niederes Carbalkoxy, gegebenenfalls durch eine oder zwei niedere Alkylgruppen substituiertes Amino oder Carbamoyl, eine Amidgruppierung, eine Imidoylgruppierung oder eine Amidingruppierung bedeuten.

3. Verbindungen nach Anspruch 1 oder 2, worin die 6-Stellung die (S)-Konfiguration aufweist, wenn $R^3$ eine von Wasserstoff verschiedene Bedeutung besitzt.

4. Verbindungen nach einem der Ansprüche 1 bis 3, worin $R^3$ niederes 1-Hydroxyalkyl bedeutet.

5. Verbindungen nach einem der Ansprüche 1 bis 4, worin $R^2$ Carboxy bedeutet.

6. Verbindungen nach einem der Ansprüche 1 bis 5, worin $R^1$ niederes Alkoxy bedeutet.

7. (4S,5S,6S)-6-[(R)-1-Hydroxyäthyl]-4-methoxy-7-oxo-1-azabicyclo[3.2.0]hept-2-en-2-carbonsäure.

8. (4R,5S,6S)-6-[(R)-1-Hydroxyäthyl]-4-methoxy-7-oxo-1-azabicyclo[3.2.0]hept-2-en-2-carbonsäure.

9. (4R,5S,6S)-6-[(R)-1-Hydroxyäthyl]-4-methoxy-3-[(S)-3-pyrrolidinylthio]-7-oxo-1-azabicyclo[3.2.0]hept-2-en-2-carbonsäure.

10. (4R,5S,6S)-6-[(R)-1-Hydroxyäthyl]-3-[[(S)-1-(1-iminoäthyl)-3-pyrrolidinyl]thio]-4-methoxy-7-oxo-1-azabicyclo[3.2.0]hept-2-en-2-carbonsäure.

11. (4R,5S,6S)-4-Aethoxy-6-[(R)-1-hydroxyäthyl]-3-[(S)-3-pyrrolidinylthio]-7-oxo-1-azabicyclo[3.2.0]hept-2-en-2-carbonsäure.

12. (4R,5S,6S)-4-Aethoxy-6-[(R)-1-hydroxyäthyl]-3-[[(S)-1-(1-iminoäthyl)-3-pyrrolidinyl]thio]-7-oxo-1-azabicyclo-[3.2.0]hept-2-en-2-carbonsäure.

13. (4R,5S,6S)-4-Aethoxy-6-[(R)-1-hydroxyäthyl]-3-(2-pyrimidinylthio)-7-oxo-1-azabicyclo[3.2.0]hept-2-en-2-carbonsäure.

14. (4R,5S,6S)-6-[(R)-1-hydroxyäthyl]-4-(1-isopentyloxy)-3-[(S)-3-pyrrolidinylthio]-7-oxo-1-azabicyclo[3.2.0]-hept-2-en-2-carbonsäure.

15. (4R,5S,6S)-6-[(R)-1-hydroxyäthyl]-4-phenoxy-3-[(S)-3-pyrrolidinylthio]-7-oxo-1-azabicyclo[3.2.0]hept-2-en-2-carbonsäure.

16. Verbindungen nach einem der Ansprüche 1 bis 15 zur Anwendung als therapeutische Wirkstoffe.

17. Verbindungen nach einem der Ansprüche 1 bis 15 zur Anwendung als Antibiotika.

18. Verfahren zur Herstellung von Verbindungen nach einem der Ansprüche 1 bis 15, dadurch gekennzeichnet, dass man

a) eine Verbindung der allgemeinen Formel

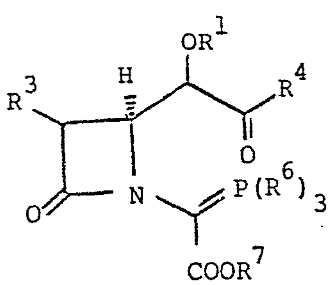
IIa

oder

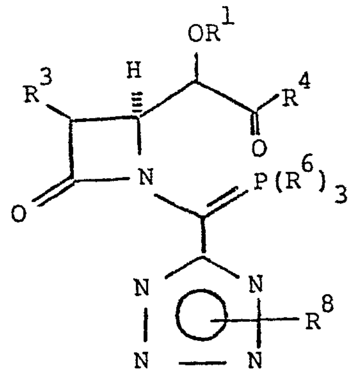
IIb

worin $R^1$, $R^3$ und $R^4$ die in Anspruch 1 angegebene Bedeutung besitzen, $R^6$ niederes Alkyl, Phenyl oder p-Methoxyphenyl, $R^7$ eine leicht abspaltbare Carboxylschutzgruppe und $R^8$ eine leicht abspaltbare N-Schutzgruppe bedeuten,

cyclisiert, oder

b) eine Verbindung der allgemeinen Formel

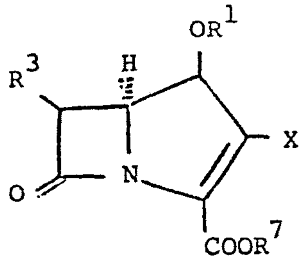

oder

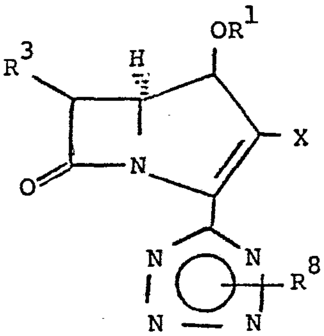

IIIb

IIIa

worin $R^1$ und $R^3$ die in Anspruch 1 angegebene und $R^7$ und $R^8$ obige Bedeutung besitzen, und X eine Abgangsgruppe bedeutet,

entweder in Gegenwart einer Base mit einem Mercaptan der allgemeinen Formel

$$HS-(A)_m-(Q)_n-(A')_p-R^5 \qquad IV$$

worin A, A', Q, $R^5$, m, n und p die in Anspruch 2 angegebene Bedeutung besitzen,

oder mit einem Salz eines solchen Mercaptans umsetzt, oder

c) eine Verbindung der allgemeinen Formel

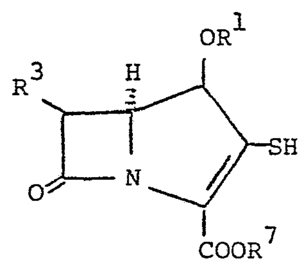

V

worin $R^1$ und $R^3$ die in Anspruch 1 angegebene und $R^7$ obige Bedeutung besitzen,

in Gegenwart einer Base oder ein Salz einer Verbindung der Formel V mit einer Verbindung der allgemeinen Formel

$$X'-(A)_m-(Q)_n-(A')_p-R^5 \qquad VI$$

worin A, A', Q, $R^5$, m, n und p die in Anspruch 2 angegebene Bedeutung besitzen, und X' eine Abgangsgruppe

bedeutet,

umsetzt, oder

d) eine Verbindung der allgemeinen Formel

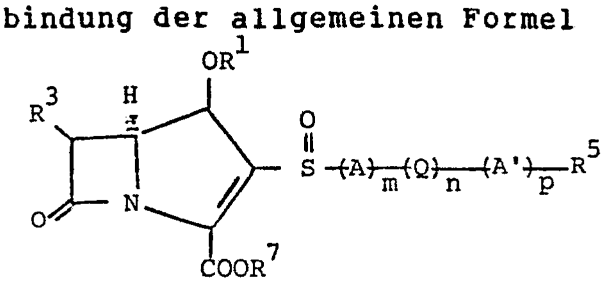

VII

worin $R^1$ und $R^3$ die in Anspruch 1 angegebene, A, A', Q, $R^5$, m, n und p die in Anspruch 2 angegebene und $R^7$ obige Bedeutung besitzen,

reduziert, worauf man erwünschtenfalls den in einer erhaltenen Verbindung allfällig vorhandenen Substituenten in 3-Stellung in an sich bekannter Weise abwandelt und/oder eine erhaltene Verbindung der Formel I entweder als einen unter physiologischen Bedingungen hydrolysierbaren Ester isoliert, oder allfällig vorhandene Schutzgruppen abspaltet und erwünschtenfalls eine so erhaltene Verbindung in ein pharmazeutisch annehmbares Salz und/oder Hydrat überführt.

19. Verbindungen der in Anspruch 18 definierten Formeln IIa, IIb, IIIa, IIIb, V und VII.

20. Verbindungen der allgemeinen Formeln

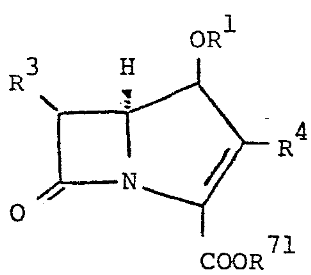

und

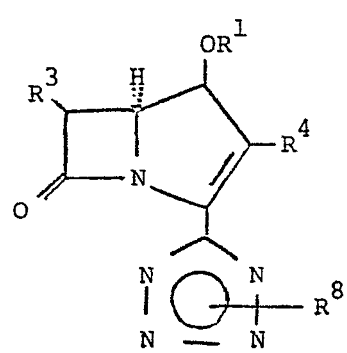

XXXb

XXXa

worin $R^1$, $R^3$, $R^4$ und $R^8$ die in den Ansprüchen 1 und 18 angegebene Bedeutung besitzen, und $R^{71}$ eine

leicht abspaltbare, jedoch unter physiologischen Bedingungen stabile Carboxylschutzgruppe bedeutet.

21. Arzneimittel, enthaltend eine Verbindung nach einem der Ansprüche 1 bis 15 und einen therapeutisch inerten Träger.

22. Antibiotisch wirksames Mittel, enthaltend eine Verbindung nach einem der Ansprüche 1 bis 15 und einen therapeutisch inerten Träger.

23. Verwendung von Verbindungen nach einem der Ansprüche 1 bis 15 bei der Bekämpfung oder Verhütung von Krankheiten.

24. Verwendung von Verbindungen nach einem der Ansprüche 1 bis 15 bei der Bekämpfung oder Verhütung von Infektionskrankheiten.

25. Verwendung von Verbindungen nach einem der Ansprüche 1 bis 15 zur Herstellung von antibiotisch wirksamen Mitteln.

***

Europäisches Patentamt

# EUROPÄISCHER RECHERCHENBERICHT

0170019
Nummer der Anmeldung

EP 85 10 7420

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| A | EP-A-0 040 494 (PFIZER INC.)<br>* Seite 4, Zeile 5 - Seite 7, Zeile 33 *<br>--- | 1,16,17 | C 07 D 487/04<br>A 61 K 31/40<br>C 07 F 9/65 //<br>C 07 D 205/08<br>C 07 D 405/04<br>C 07 F 7/10<br>(C 07 D 487/04<br>C 07 D 209:00<br>C 07 D 205:00 ) |
| A | EP-A-0 045 198 (TAKEDA CHEMICAL INDUSTRIES LTD.)<br>* Seite 2, Zeile 4 - Seite 10, Zeile 13 *<br>--- | 1-5,16-18,21-25 | |
| A | EP-A-0 089 139 (BEECHAM GROUP PLC)<br>* Seite 1, Zeile 10 - Seite 11, Zeile 6 *<br>--- | 1-5,16-18,21-25 | |
| A | EP-A-0 072 710 (SANKYO CO. LTD.)<br>* Seite 3, Zeile 13 - Seite 17, Zeile 15 *<br>--- | 1-5,16-18,21-25 | RECHERCHIERTE SACHGEBIETE (Int. Cl.4)<br>C 07 D 487/00<br>C 07 F 9/00<br>A 61 K 31/00 |
| A | EP-A-0 102 239 (SANKYO CO. LTD.)<br>* Seite 5, Zeile 16, Seite 28, Zeile 24 *<br>----- | 1,18 | |

Der vorliegende Recherchenbericht wurde für alle Patentansprüche erstellt.

| Recherchenort | Abschlußdatum der Recherche | Prüfer |
|---|---|---|
| BERLIN | 23-09-1985 | VAN AMSTERDAM L.J.P. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument